# FASCICULE DE BREVET EUROPEEN

(11) **EP 3 344 763 B1**
(45) Date de publication et mention de la délivrance du brevet: **06.10.2021**
(21) Numéro de dépôt: 16770052.5
(22) Date de dépôt: 23.08.2016
(51) Int. Cl.: C12N 9/34, C12R 1/865, C07K 14/395, C12N 1/18, C12N 15/81, C12P 7/06

(54) **SOUCHES DE LEVURE CO-EXPRIMANT DES GLUCOAMYLASES EXOGENES, LEUR PROCEDE D'OBTENTION ET LEUR UTILISATION POUR PRODUIRE DU BIOETHANOL**
HEFESTÄMME MIT KOEXPRESSION EXOGENER GLUCOAMYLASEN, VERFAHREN ZUR GEWINNUNG DIESER HEFESTÄMME UND DEREN VERWENDUNG ZUR HERSTELLUNG VON BIOETHANOL
YEAST STRAINS CO-EXPRESSING EXOGENOUS GLUCOAMYLASES, THE METHOD FOR OBTAINING SAID YEAST STRAINS AND THE USE THEREOF TO PRODUCE BIOETHANOL

(30) Priorité: 31.08.2015 FR 1558079
(43) Date de publication de la demande: 11.07.2018
(73) Titulaire: Lesaffre et Compagnie, 75001 Paris (FR)
(72) Inventeur: PETIT, Maud, 59350 Saint-André-lez-Lille (FR); PIGNEDE, Georges, 59700 Marcq en Baroeul (FR); BAVOUZET, Jean-Michel, 60200 Compiègne (FR); THOMAS, Benoît, 59700 MARCQ EN BAROEUL (FR)
(74) Mandataire: Plasseraud IP
(86) Numéro de dépôt international: PCT/FR2016/052107
(87) Numéro de publication internationale: WO 2017/037362

(56) Documents cités:
- FAVARO L ET AL: "Consolidated bioprocessing of starchy substrates into ethanol by industrial Saccharomyces cerevisiae strains secreting fungal amylases", BIOTECHNOLOGY AND BIOENGINEERING 20150901 JOHN WILEY AND SONS INC. USA, vol. 112, no. 9, 14 juillet 2015 (2015-07-14), pages 1751-1760, XP002754198, ISSN: 0006-3592
- MARKO J VIKTOR ET AL: "Raw starch conversion by Saccharomyces cerevisiae expressing Aspergillus tubingensis amylases", BIOTECHNOLOGY FOR BIOFUELS, BIOMED CENTRAL LTD, GB, vol. 6, no. 1, 29 novembre 2013 (2013-11-29), page 167, XP021170405, ISSN: 1754-6834, DOI: 10.1186/1754-6834-6-167
- JI-HYE KIM ET AL: "Construction of a direct starch-fermenting industrial strain of Saccharomyces cerevisiae producing glucoamylase, [alpha]-amylase and debranching enzyme", BIOTECHNOLOGY LETTERS SPRINGER SCIENCE+BUSINESS MEDIA B.V. NETHERLANDS, vol. 32, no. 5, mai 2010 (2010-05), pages 713-719, XP002754199, ISSN: 0141-5492
- LORENA LATORRE-GARC PRG A-A ET AL: "Overexpression of the glucoamylase-encoding STA1 gene of Saccharomyces cerevisiae var. diastaticus in laboratory and industrial strains of Saccharomyces", WORLD JOURNAL OF MICROBIOLOGY AND BIOTECHNOLOGY, KLUWER ACADEMIC PUBLISHERS, DO, vol. 24, no. 12, 15 août 2008 (2008-08-15) , pages 2957-2963, XP019650407, ISSN: 1573-0972, DOI: 10.1007/S11274-008-9837-9

## Description

### Domaine technique de l'invention

La présente invention concerne des souches de levure de *Saccharomyces cerevisiae* génétiquement modifiées de manière à ce qu'elles co-expriment des gènes codants pour des glucoamylases d'origine fongique et de *Saccharomyces cerevisiae* var. *diastaticus.* De telles souches trouvent particulièrement un intérêt dans la production de biocarburant, notamment de bioéthanol. La présente invention concerne également un procédé d'obtention de ces levures ainsi que l'utilisation de ces levures pour produire du bioéthanol.

### Arrière-plan technologique

L'utilisation de la biomasse pour la production de bioéthanol a suscité un intérêt considérable ces dernières années. L'éthanol produit à partir de résidus agricoles, de déchets industriels et de plantes à croissance rapide a notamment été proposé comme carburant alternatif prometteur.

Actuellement, le bioéthanol dit de première génération est produit principalement à partir de sucre de canne et de grains riches en amidon au Brésil et aux Etats-Unis respectivement, en utilisant des souches de levure *Saccharomyces cerevisiae,* qui permettent de fermenter le glucose en éthanol avec un titre alcoolique, une productivité et un rendement élevé.

Le processus permettant la conversion d'amidon en bioéthanol nécessite une préhydrolyse et une liquéfaction de l'amidon de la biomasse, la conversion de l'amidon liquéfié en sucres fermentescibles (par hydrolyse de l'amidon) et la fermentation de ces sucres en éthanol. Ces deux dernières étapes sont souvent réalisées de façon simultanée.

L'hydrolyse de l'amidon nécessite l'action d'enzymes dites amylolytiques, mais, malheureusement, la majorité des *S. cerevisiae* est dépourvue de ce type d'enzymes. Actuellement, la production d'éthanol à partir de la biomasse composée d'amidon requiert par conséquent l'addition d'enzymes exogènes en deux étapes : une première étape d'addition d'enzymes amylolytiques exogènes de manière à préhydrolyser et liquéfier l'amidon contenu dans la biomasse; et une deuxième étape où l'on utilise d'autres enzymes exogènes pour hydrolyser l'amidon liquéfié et une souche de levure de *S. cerevisiae* pour fermenter les sucres fermentescibles libérés.

L'utilisation d'enzymes exogènes engendre une augmentation des coûts et des pertes de temps non négligeables, et il serait donc très avantageux d'obtenir des souches de levures qui soient à la fois capables d'hydrolyser l'amidon liquéfié tout en étant capable de fermenter de manière efficace les sucres issus de l'hydrolyse de l'amidon liquéfié.

### Invention

Dans ce cadre, les inventeurs de la présente invention ont développé une souche génétiquement modifiée de *Saccharomyces cerevisiae,* ladite souche co-exprimant plusieurs gènes de glucoamylases exogènes. En particulier les souches de *Saccharomyces cerevisiae* selon l'invention co-expriment à la fois un gène codant une glucoamylase d'origine fongique ainsi qu'un gène codant la glucoamylase de *Saccharomyces cerevisiae* var. *diastaticus.* Les inventeurs ont démontré que ces souches étaient capables d'hydrolyser l'amidon liquéfié extrait de la biomasse tout en réussissant à fermenter efficacement les sucres issus de cette hydrolyse. En effet, l'utilisation d'une souche de levure selon la présente invention permet de remplacer tout ou partie de la quantité d'enzymes exogènes nécessaires lors de la conversion de l'amidon liquéfié en bioéthanol.

Ainsi, selon un premier aspect, la présente invention concerne une souche de levure de *Saccharomyces cerevisiae,* caractérisée en ce qu'elle co-exprime :
- un gène codant une glucoamylase d'origine fongique choisie parmi une glucoamylase *d'Aspergillus niger* et une glucoamylase de *Saccharomycopsis fibuligera;* et
- un gène codant la glucoamylase de *Saccharomyces cerevisiae* var. *diastaticus,*
où le gène codant la glucoamylase *d'Aspergillus niger* a la séquence nucléique SEQ ID NO : 1, le gène codant la glucoamylase de *Saccharomycopsis fibuligera* a la séquence nucléique SEQ ID NO : 17, et le gène codant la glucoamylase de *Saccharomyces cerevisiae var. diastaticus* a la séquence nucléique SEQ ID NO : 3,
et en ce qu'elle contient :
- entre 2 et 10 copies, bornes incluses, du gène codant la glucoamylase d'origine fongique; et
- entre 2 et 10 copies, bornes incluses, du gène codant la glucoamylase de *Saccharomyces cerevisiae* var. *diastaticus.*

Les inventeurs ont également mis au point une méthode permettant d'obtenir des souches de *Saccharomyces cerevisiae* ayant la capacité à la fois d'hydrolyser l'amidon et de fermenter les sucres issus de cette hydrolyse.

Ainsi, selon un second aspect, la présente invention concerne une méthode d'obtention d'une souche de levure, ladite méthode comprenant les étapes suivantes :
a) modification génétique d'une levure de *Saccharomyces cerevisiae* de manière à la faire co-exprimer un gène codant une glucoamylase d'origine fongique choisie parmi une glucoamylase *d'Aspergillus niger* et une glucoamylase de *Saccharomycopsis fibuligera* et un gène codant la glucoamylase de *Saccharomyces cerevisiae* var. *diastaticus* et de manière à ce qu'elle contienne entre 2 et 10 copies, bornes incluses, du gène codant la glucoamylase d'origine fongique; et entre 2 et 10 copies, bornes incluses, du gène codant la glucoamylase de *Saccharomyces cerevisiae* var. *diastaticus*;
b) mise en culture et fermentation de la souche obtenue à l'étape a) sur un milieu dextrine ;
c) sélection des souches présentant une cinétique de fermentation au moins égale ou supérieure à celle obtenue avec la souche déposée le 9 Juillet 2015 en vertu du traité de Budapest à la CNCM sous le numéro I-4999 dans les mêmes conditions,
   dans laquelle le gène codant la glucoamylase *d'Aspergillus niger* a la séquence nucléique SEQ ID NO : 1, le gène codant la glucoamylase de *Saccharomyces fibuligera* a la séquence nucléique SEQ ID NO : 17, et le gène codant la glucoamylase de *Saccharomyces cerevisiae var. diastaticus* a la séquence nucléique SEQ ID NO : 3.

Selon un autre aspect, la présente invention concerne un procédé de production de bioéthanol à partir d'une biomasse caractérisé en ce qu'il comprend les étapes suivantes :
a) préhydrolyse et liquéfaction de l'amidon de la biomasse ;
b) mise en contact de l'amidon liquéfié obtenu à l'étape a) avec une levure de *Saccharomyces cerevisiae* telle que décrite dans l'une quelconque des revendications 1 à 9;
c) hydrolyse et fermentation de l'amidon liquéfié par ladite levure ;
d) extraction de l'éthanol produit à l'étape c).

En outre, la présente invention concerne l'utilisation d'une souche de levure *Saccharomyces cerevisiae* telle que décrite dans l'une quelconque des revendications 1 à 9 pour la production de biocarburant.

### Description détaillée de l'invention

Afin d'obtenir une souche de levure qui puisse hydrolyser l'amidon et fermenter les sucres issus de l'hydrolyse de l'amidon, les inventeurs ont génétiquement modifié une souche de *Saccharomyces cerevisiae* de manière à lui faire co-exprimer deux gènes codant pour des glucoamylases exogènes, comme indiqué ci-dessus.

Ainsi, un premier objet de la présente invention est une souche de levure de *Saccharomyces cerevisiae,* caractérisée en ce qu'elle co-exprime :
- un gène codant une glucoamylase d'origine fongique choisie parmi une glucoamylase *d'Aspergillus niger* et une glucoamylase de *Saccharomycopsis fibuligera*; et
- un gène codant la glucoamylase de *Saccharomyces cerevisiae* var. *diastaticus,*
   où le gène codant la glucoamylase *d'Aspergillus niger* a la séquence nucléique SEQ ID NO : 1, le gène codant la glucoamylase de *Saccharomycopsis fibuligera* a la séquence nucléique SEQ ID NO : 17, et le gène codant la glucoamylase de *Saccharomyces cerevisiae var. diastaticus* a la séquence nucléique SEQ ID NO : 3,
   et en ce qu'elle contient :
   - entre 2 et 10 copies, bornes incluses, du gène codant la glucoamylase d'origine fongique; et
   - entre 2 et 10 copies, bornes incluses, du gène codant la glucoamylase de *Saccharomyces cerevisiae* var. *diastaticus.*

Particulièrement la présente invention a pour objet une telle souche de levure de *Saccharomyces cerevisiae,* caractérisée en ce que la glucoamylase de *Saccharomyces cerevisiae* var. *diastaticus* a la séquence protéique SEQ ID NO : 4.

De manière surprenante, les inventeurs ont découvert que l'utilisation spécifique d'un gène de glucoamylase de *Saccharomyces cerevisiae* var. *diastaticus* et d'un gène de glucoamylase d'origine fongique tel que défini ci-dessus permettait d'obtenir des souches avec d'excellentes capacités d'hydrolyse.

Ces résultats sont particulièrement étonnants, car la glucoamylase de *Saccharomyces cerevisiae* var. *diastaticus* est connue pour avoir un rendement beaucoup moins efficace que celui obtenu avec les glucoamylases d'origine fongique, ce qui en fait une enzyme très peu utilisée par les enzymiers. Ce postulat est par exemple démontré dans la demande de brevet internationale publiée sous la référence WO2011/153516, qui décrit le criblage enzymes dont la glucoamylase de *Saccharomyces cerevisiae* var. *diastaticus* (GenBank ID : AAA35107.1). Dans ce document, la glucoamylase de *Saccharomyces cerevisiae* var. *diastaticus* n'est pas retenue comme étant intéressante pour son activité enzymatique.

L'expression « souche de levure » désigne une population relativement homogène de cellules de levure. Une souche de levure est obtenue à partir d'un clone, un clone étant une population de cellules obtenue à partir d'une seule cellule de levure.

Par « gène codant la glucoamylase» on entend ici une séquence d'acides aminés qui, lorsqu'elle est exprimée, va donner une protéine de glucoamylase fonctionnelle.

Par « glucoamylase » on entend ici une enzyme capable d'hydrolyser les liaisons glycosidiques α-1,4 de l'amidon brut ou soluble à partir de l'extrémité non réductrice de l'amylose et de l'amylopectine. Les amylases sont également connues sous la dénomination d'amyloglucosidases ou de γ-amylases (MEDLINE référence : EC 3.2.1.3). En plus d'agir sur les liaisons α-1,4, l'enzyme glucoamylase peut hydrolyser lentement les liaisons α-1,6 des molécules d'amylopectine, à condition que la liaison voisine dans la séquence soit une liaison α-1,4.

Une glucoamylase d'origine fongique peut être une des glucoamylases commerciales connues pour leur bonne activité enzymatique et, en particulier, la glucoamylase d'origine fongique est sélectionnée dans le groupe consistant en : une glucoamylase *d'Aspergillus niger,* une glucoamylase de *Saccharomycopsis fibuligera,* une glucoamylase de *Trichoderma reesei,* une glucoamylase de *Rhizopus oryzae,* une glucoamylase *d'Aspergillus oryzae* et une glucoamylase *Thermomyces lanuginosis.*

Ces glucoamylases sont connues de l'homme du métier, et leurs séquences sont accessibles sous les références GenBank (http://www.ncbi.nlm.nih.gov/genbank/) suivantes : *Trichoderma reesei (ETS06561), Rhizopus oryzae (BAA00033), Aspergillus oryzae (BAA00841), Thermomyces lanuginosis (ABQ23180).*

Selon un mode de réalisation particulier de la présente invention, la glucoamylase d'origine fongique est une glucoamylase *d'Aspergillus niger* ou de *Saccharomycopsis fibuligera.*

La glucoamylase de *Saccharomycopsis fibuligera* est codée par le gène *GLU0111* qui présente la séquence nucléique correspondant à la SEQ ID NO : 17 et a pour séquence protéique la séquence correspondant à la SEQ ID NO : 18. La glucoamylase *d'Aspergillus niger* est codée par le gène *GLAA* qui présente la séquence nucléique correspondant à la SEQ ID NO : 1 et a pour séquence protéique la séquence correspondant à la SEQ ID NO : 2.

La glucoamylase de *Saccharomyces cerevisiae* var. *diastaticus* est codée par le gène *STA1* qui présente la séquence nucléique correspondant à la SEQ ID NO : 3 et a pour séquence protéique la séquence correspondant à la SEQ ID NO : 4.

Ainsi, dans un mode de réalisation particulier, la présente invention a pour objet une souche de levure de *Saccharomyces cerevisiae* telle que définie ci-dessus, caractérisée en ce qu'elle contient la séquence nucléique SEQ ID NO : 1 et la séquence nucléique SEQ ID NO : 3.

Dans un mode de réalisation, la présente invention a pour objet une souche de levure de *Saccharomyces cerevisiae* telle que définie ci-dessus, caractérisée en ce qu'elle co-exprime :
- un gène codant la glucoamylase *d'Aspergillus niger;* et
- un gène codant la glucoamylase de *Saccharomyces cerevisiae* var. *diastaticus.*

Dans mode de réalisation particulier, l'invention concerne une souche de levure de *Saccharomyces cerevisiae* telle que définie ci-dessus, caractérisée en ce qu'elle co-exprime :
- un gène codant la glucoamylase *d'Aspergillus niger;* et
- un gène codant la glucoamylase de *Saccharomyces cerevisiae* var. *diastaticus*
où la glucoamylase de *Saccharomyces cerevisiae* var. *diastaticus* a la séquence protéique SEQ ID NO : 4 et la glucoamylase *d'Aspergillus niger* a la séquence protéique SEQ ID NO : 2.

Les expressions « glucoamylase d'origine fongique» et « glucoamylase de *Saccharomyces cerevisiae* var. *diastaticus* » ne doivent pas être interprétées de manière stricte : elles englobent les glucoamylases d'origine fongique et de *Saccharomyces cerevisiae* var. *diastaticus* qui sont codées par les séquences nucléiques telles que décrites ci-dessus, mais également les variants fonctionnels de ces glucoamylases.

Typiquement, un variant fonctionnel d'une glucoamylase selon l'invention a une séquence protéique présentant un pourcentage d'identité d'au moins 80%, 90%, 95%, plus particulièrement de 99% avec la séquence protéique de ladite glucoamylase. Par exemple, les variants fonctionnels des glucoamylases *d'Aspergillus niger* et de *Saccharomyces cerevisiae* var. *diastaticus* ont une séquence protéique présentant un pourcentage d'identité d'au moins 80%, 90%, 95%, plus particulièrement de 99% respectivement avec la séquence SEQ ID NO: 2 ou 4.

Le « pourcentage d'identité » est une comparaison entre des séquences d'acides aminés, et est déterminé en comparant deux séquences alignées de manière optimale sur une fenêtre de comparaison. L'homme du métier sait comment calculer un pourcentage d'identité entre deux séquences et dispose de nombreux outils le lui permettant. L'une des deux séquences peut présenter des insertions, substitutions et des délétions d'acides aminés par rapport à l'autre séquence.

L'homme du métier saura comment sélectionner des variants fonctionnels des glucoamylases selon l'invention. Par « variant fonctionnel » on entend un variant qui conserve son activité de glucoamylase et ce avec des caractéristiques de cinétique d'hydrolyse de l'amidon similaires. Des méthodes permettant de mesurer et de comparer des cinétiques d'hydrolyse de l'amidon sont décrites dans la partie expérimentale de la présente demande. Des variants fonctionnels peuvent être préparés par diverses méthodes conventionnelles, telles que par exemple la mutagénèse aléatoire ou la mutagénèse dirigée.

L'homme du métier connait de multiples méthodes permettant d'introduire un gène dans une souche de levure, notamment via l'utilisation de vecteurs comprenant des cassettes d'expression. On entend par « vecteur » toute séquence d'ADN dans laquelle il est possible d'insérer des fragments d'acides nucléiques étrangers, les vecteurs permettant d'introduire de l'ADN étranger dans une cellule hôte. Des exemples de vecteurs sont les plasmides, les cosmides, les vecteurs dérivés de virus. Les vecteurs permettent soit l'intégration des gènes hétérologues directement dans le génome de la levure, soit leur expression dans un plasmide indépendant.

L'introduction de vecteurs dans une cellule hôte est un procédé largement connu de l'homme du métier. Plusieurs méthodes sont notamment décrites dans « Current Protocols in Molecular Biology », 13.7.1-13.7.10 ; ou encore dans Ellis T. et al., Integrative Biology, 2011, 3(2), 109-118.

Selon l'invention, le gène codant une glucoamylase d'origine fongique et celui codant la glucoamylase de *Saccharomyces cerevisiae* var. *diastaticus* peuvent être insérés au sein d'un seul et même vecteur, ou au sein de deux vecteurs séparés.

Ainsi, selon un aspect particulier de l'invention, le gène codant une glucoamylase d'origine fongique et celui codant la glucoamylase de *Saccharomyces cerevisiae* var. *diastaticus* sont intégrés séparément chacun dans un vecteur. Selon un mode de réalisation particulier, le vecteur est un plasmide.

Dans un mode de réalisation particulier de l'invention, le gène codant une glucoamylase d'origine fongique et celui codant la glucoamylase de *Saccharomyces cerevisiae* var. *diastaticus* sont intégrés au sein du génome de ladite levure.

Le vecteur selon l'invention pourra également porter un marqueur de sélection. On entend par « marqueur de sélection » un gène dont l'expression confère aux levures qui le contiennent une caractéristique permettant de les sélectionner. Il s'agit par exemple d'un gène de résistance aux antibiotiques ou d'un gène permettant la croissance de la levure sur un milieu particulier.

Les gènes selon l'invention peuvent être liés opérationnellement à un promoteur, un terminateur ou toute autre séquence nécessaire à son expression dans levure.

Dans un mode particulier de l'invention, l'expression des gènes codant pour les glucoamylases d'origines fongiques et de *Saccharomyces cerevisiae* var. *diastaticus* est contrôlée par un promoteur dit « fort ». L'homme du métier connait la signification de promoteur fort. Un promoteur fort est par exemple le promoteur *pADH1,* le promoteur *pTEF,* ou le promoteur *pTDH3.*

Ainsi, dans un mode de réalisation, la présente invention concerne une souche de levure de *Saccharomyces cerevisiae* telle que décrite ci-dessus, dans laquelle l'expression du gène codant une glucoamylase d'origine fongique et celui codant la glucoamylase de *Saccharomyces cerevisiae* var. *diastaticus* est contrôlée par le promoteur *pADH1.*

Les gènes codant pour les glucoamylases d'origines fongiques et de *Saccharomyces cerevisiae* var. *diastaticus* peuvent être présents en plusieurs copies.

Ainsi, la présente invention concerne une souche de levure de *Saccharomyces cerevisiae* telle que décrite ci-dessus, caractérisée en ce qu'elle comprend m copies du gène codant une glucoamylase d'origine fongique et n copies du gène codant la glucoamylase de *Saccharomyces cerevisiae* var. *diastaticus,* où m est un nombre entier compris entre 2 et 10 et n est un nombre entier compris entre 2 et 10.

m et n sont donc indépendamment égaux à 2, 3, 4, 5, 6, 7, 8, 9 ou 10.

Dans un mode de réalisation plus particulier, m est un nombre entier compris entre 2 et 8, et n est un nombre entier compris entre 2 et 8.

L'invention concerne particulièrement les souches de levures de *Saccharomyces cerevisiae* telles que décrites ci-dessus, lesdites souches étant la souche déposée le 6 août 2015 en vertu du traité de Budapest à la CNCM sous le numéro I-5005 ou la souche déposée le 9 Juillet 2015 en vertu du traité de Budapest à la CNCM sous le numéro I-4997.

Les souches de levure I-5005 et I-4997 comprennent au moins 4 copies du gène codant la glucoamylase *d'Aspergillus niger* et au moins 3 copies du gène codant la glucoamylase de *Saccharomyces cerevisiae* var. *diastaticus.*

L'invention concerne également deux souches de levures de *Saccharomyces cerevisiae* telles que décrites ci-dessus, lesdites souches étant la souche déposée le 11 Août 2016 en vertu du traité de Budapest à la CNCM sous le numéro I-5119 ou la souche déposée le 11 Août 2016 en vertu du traité de Budapest à la CNCM sous le numéro I-5120.

La souche de levure I-5119 comprend 8 copies du gène codant la glucoamylase *d'Aspergillus niger* et 4 copies du gène codant la glucoamylase de *Saccharomyces cerevisiae* var. *diastaticus.*

La souche de levure I-5120 comprend 4 copies du gène codant la glucoamylase *d'Aspergillus niger* et 8 copies du gène codant la glucoamylase de *Saccharomyces cerevisiae* var. *diastaticus.*

L'invention concerne également deux souches de levures de *Saccharomyces cerevisiae* telles que décrites ci-dessus, lesdites souches étant la souche déposée le 11 Août 2016 en vertu du traité de Budapest à la CNCM sous le numéro I-5121 ou la souche déposée le 11 Août 2016 en vertu du traité de Budapest à la CNCM sous le numéro I-5122.

La souche de levure I-5121 comprend 4 copies du gène codant la glucoamylase de *Saccharomycopsis fibuligera* et 4 copies du gène codant la glucoamylase de *Saccharomyces cerevisiae* var. *diastaticus.*

La souche de levure I-5122 comprend 4 copies du gène codant la glucoamylase de *Saccharomycopsis fibuligera* et 8 copies du gène codant la glucoamylase de *Saccharomyces cerevisiae* var. *diastaticus.*

Les inventeurs ont parallèlement mis au point une méthode permettant d'obtenir des souches de *Saccharomyces cerevisiae* qui sont capables d'hydrolyser l'amidon.

Ainsi, selon un autre aspect, la présente invention a pour objet une méthode d'obtention d'une souche de levure, ladite méthode comprenant les étapes suivantes :
a) modification génétique d'une levure de *Saccharomyces cerevisiae* de manière à la faire co-exprimer un gène codant une glucoamylase d'origine fongique choisie parmi une glucoamylase *d'Aspergillus niger* et une glucoamylase de *Saccharomycopsis fibuligera* et un gène codant la glucoamylase de *Saccharomyces cerevisiae* var. *diastaticus* et de manière à ce qu'elle contienne entre 2 et 10 copies, bornes incluses, du gène codant la glucoamylase d'origine fongique; et entre 2 et 10 copies, bornes incluses, du gène codant la glucoamylase de *Saccharomyces cerevisiae* var. *diastaticus;*
b) mise en culture et fermentation de la souche obtenue à l'étape a) sur un milieu dextrine ;
c) sélection des souches présentant une cinétique de fermentation au moins égale ou supérieure à celle obtenue avec la souche déposée le 9 Juillet 2015 en vertu du traité de Budapest à la CNCM sous le numéro I-4999 dans les mêmes conditions,
dans laquelle le gène codant la glucoamylase *d'Aspergillus niger* a la séquence nucléique SEQ ID NO : 1, le gène codant la glucoamylase de *Saccharomyces fibuligera* a la séquence nucléique SEQ ID NO : 17, et le gène codant la glucoamylase de *Saccharomyces cerevisiae var. diastaticus* a la séquence nucléique SEQ ID NO : 3.

La levure *Saccharomyces cerevisiae* de l'étape a) est une levure utilisée pour la production de bioéthanol.

Selon un mode de réalisation particulier de l'invention, la levure *Saccharomyces cerevisiae* de l'étape a) est la levure Ethanol Red^{®}, ci-après nommée ER, déposée à la CNCM le 4 septembre 2008 sous le numéro I-4071.

Par « milieu dextrine », on entend un milieu synthétique contenant des dextrines tel que connu de l'homme du métier. Il s'agit par exemple d'un milieu synthétique contenant des dextrines d'amidon (220 g/kg), de l'extrait de levure (5 g/kg), de l'urée (2 g/kg), du dihydrogénophosphate de potassium (1 g/kg) ainsi que des minéraux et des vitamines.

La souche de référence I-4999 correspond à la souche de levure Ethanol Red^{®} génétiquement modifiée comprenant 4 copies du gène codant la glucoamylase de *Saccharomycopsis fibuligera.*

La cinétique de fermentation peut être facilement mesurée par différentes techniques connues de l'homme du métier. Par exemple, la cinétique de fermentation peut-être mesurée via un suivi de fermentation par pesée au cours du temps.

Les souches ainsi sélectionnées sont particulièrement intéressantes pour produire du biocarburant, notamment du bioéthanol à partir de biomasse.

Le terme « biomasse » désigne un ensemble de matières organiques qui peuvent être transformées en énergie. De nombreux types de biomasse, y compris le bois, les résidus agricoles, les cultures herbacées peuvent être utilisées pour la production de biocarburant, particulièrement de bioéthanol. Le bioéthanol est caractérisé de « bio » car il est produit à partir de biomasse renouvelable.

Ainsi, la présente invention concerne un procédé de production de bioéthanol à partir d'une biomasse caractérisé en ce qu'il comprend les étapes suivantes :
a) préhydrolyse et liquéfaction de l'amidon de la biomasse ;
b) mise en contact de l'amidon liquéfié obtenu à l'étape a) avec une levure selon l'invention ;
c) hydrolyse et fermentation de l'amidon liquéfié par ladite levure ;
d) extraction de l'éthanol produit à l'étape c).

Selon un mode de réalisation particulier, le procédé de production de bioéthanol décrit ci-dessus comprend en outre une étape b') d'ajout d'enzymes glucoamylases exogènes après l'étape b) et/ou au cours de l'étape c).

L'éthanol ainsi produit peut avoir de multiples utilisations, notamment dans l'industrie automobile.

L'invention concerne également l'utilisation d'une souche de levure selon l'invention telle que décrite ci-dessus pour la production de biocarburant, particulièrement de bioéthanol.

### Brève description des figures

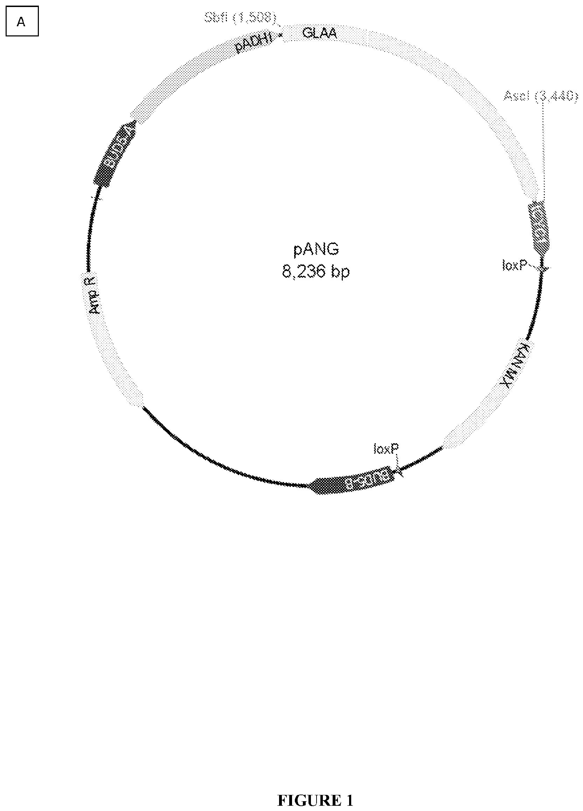

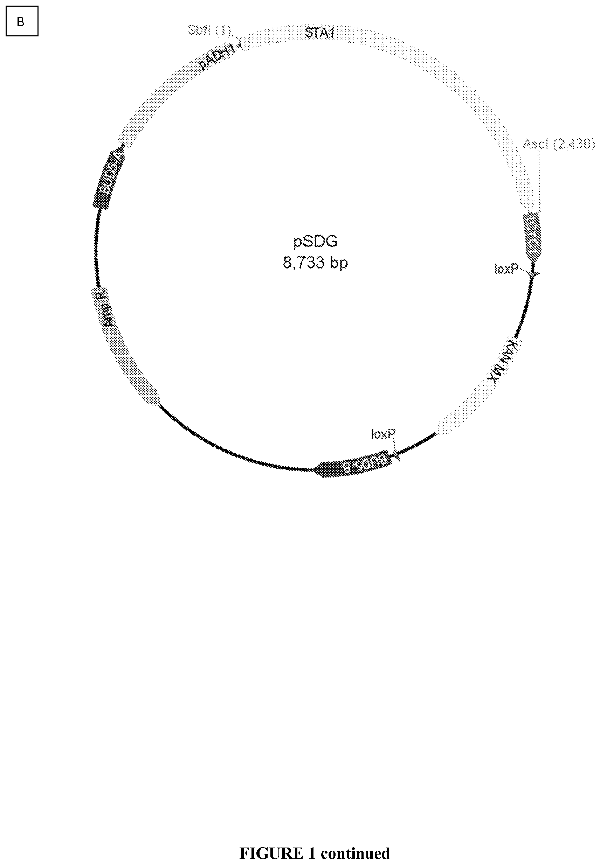

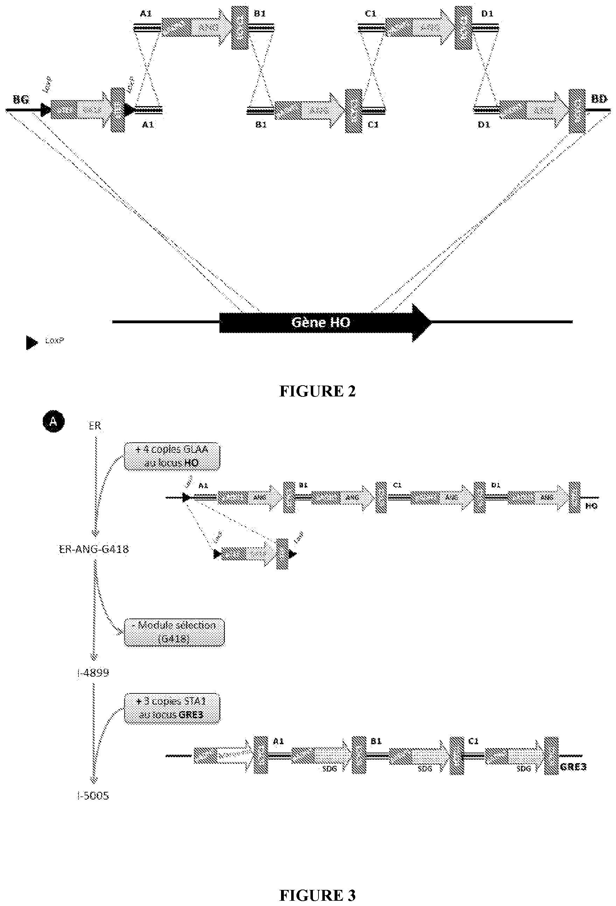

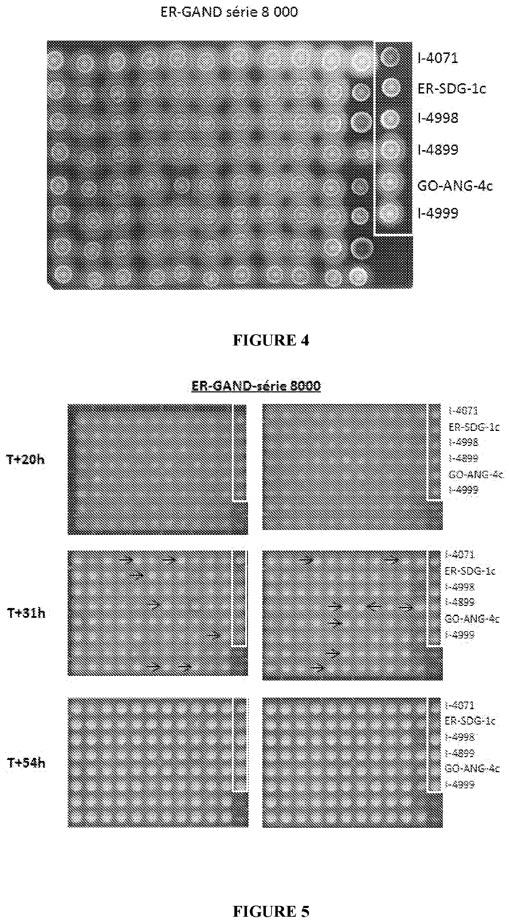

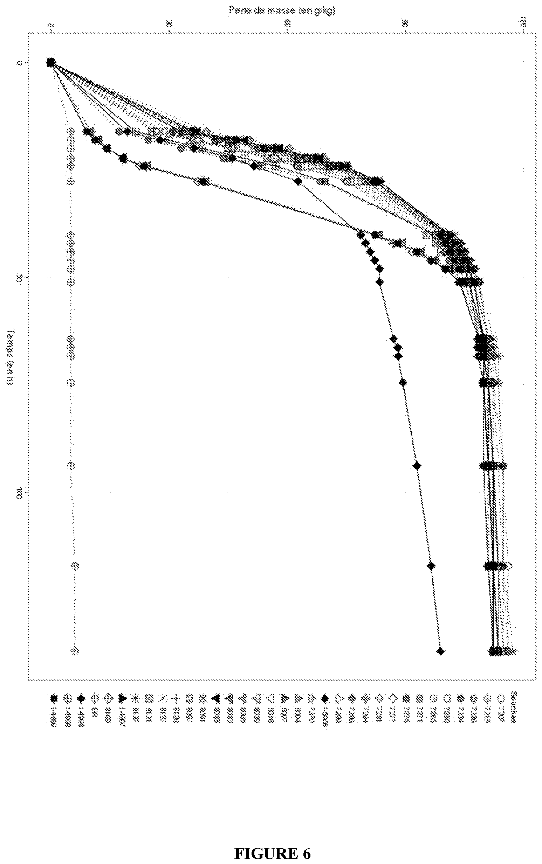

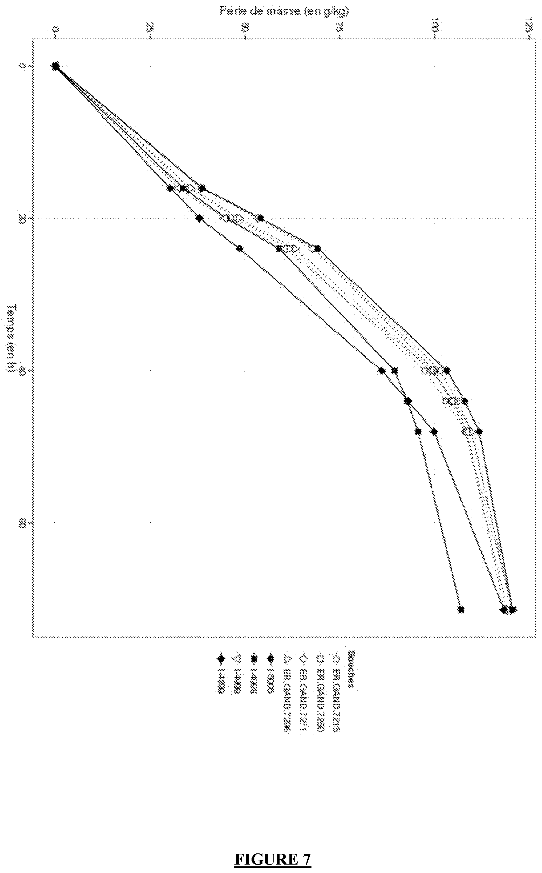

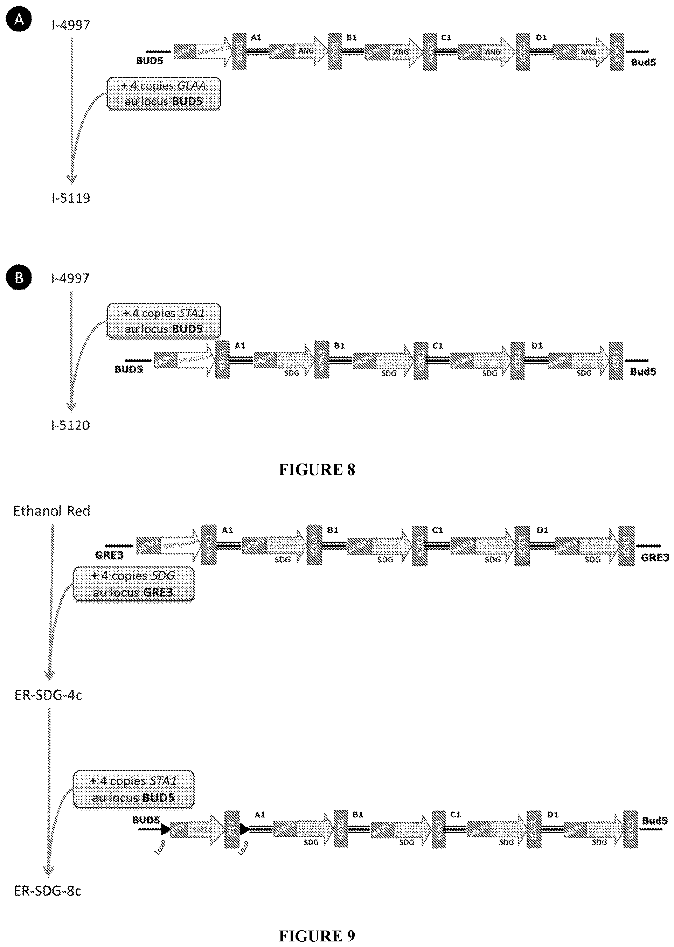

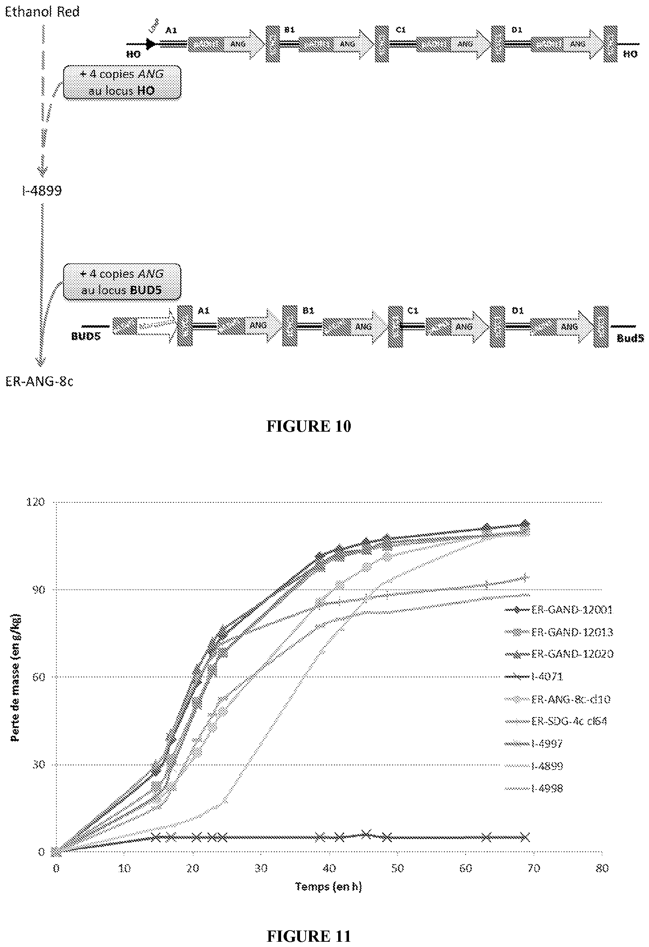

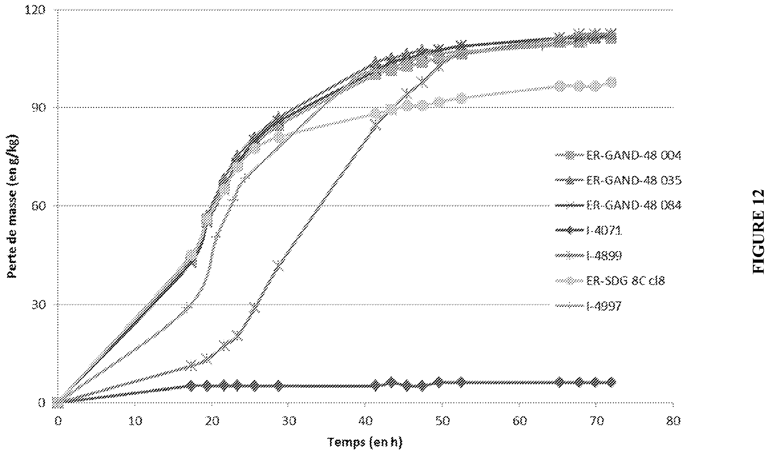

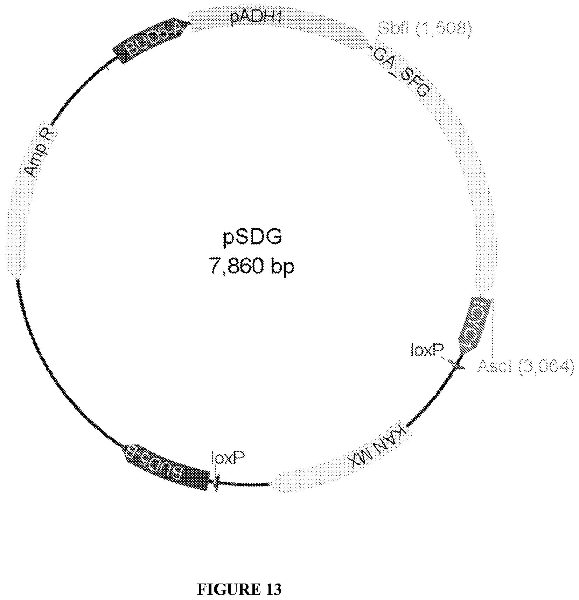

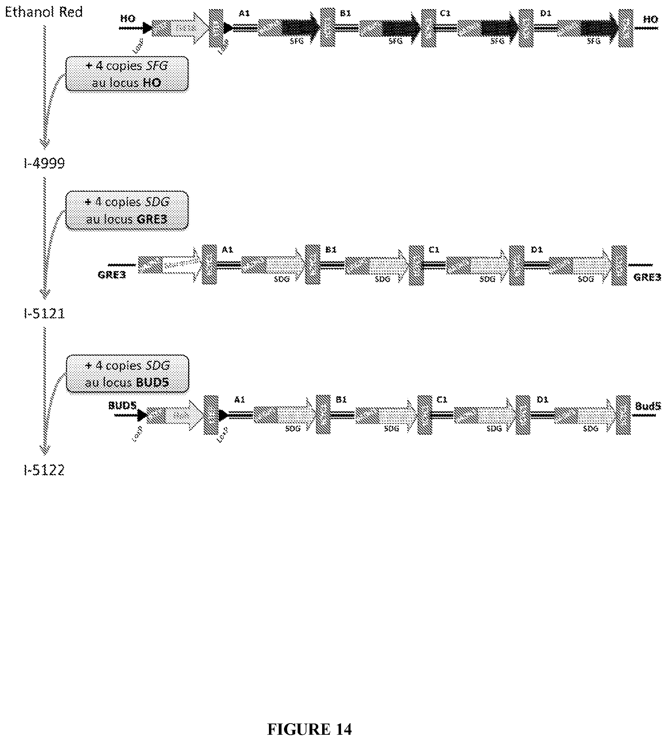

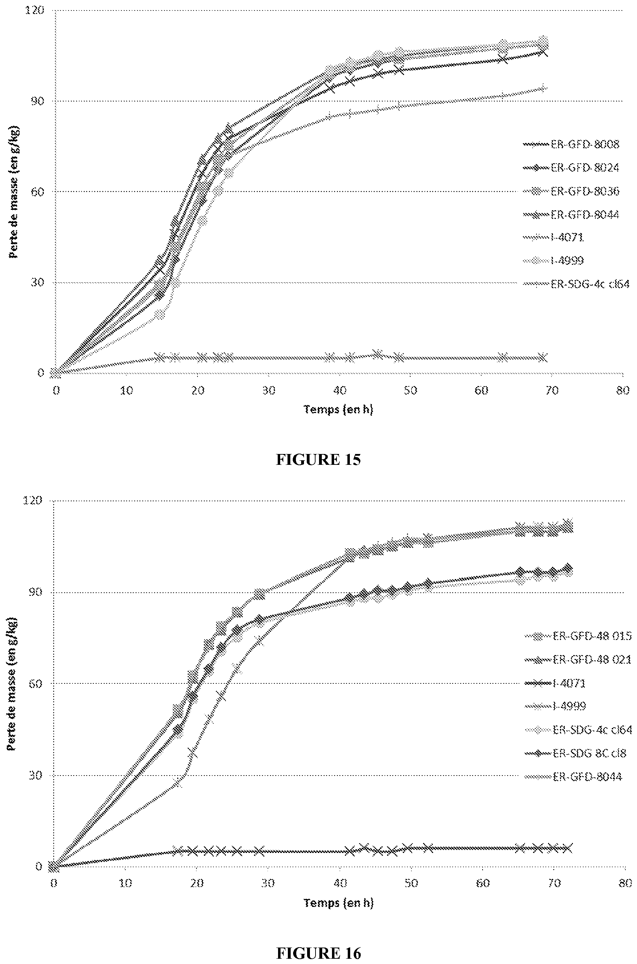

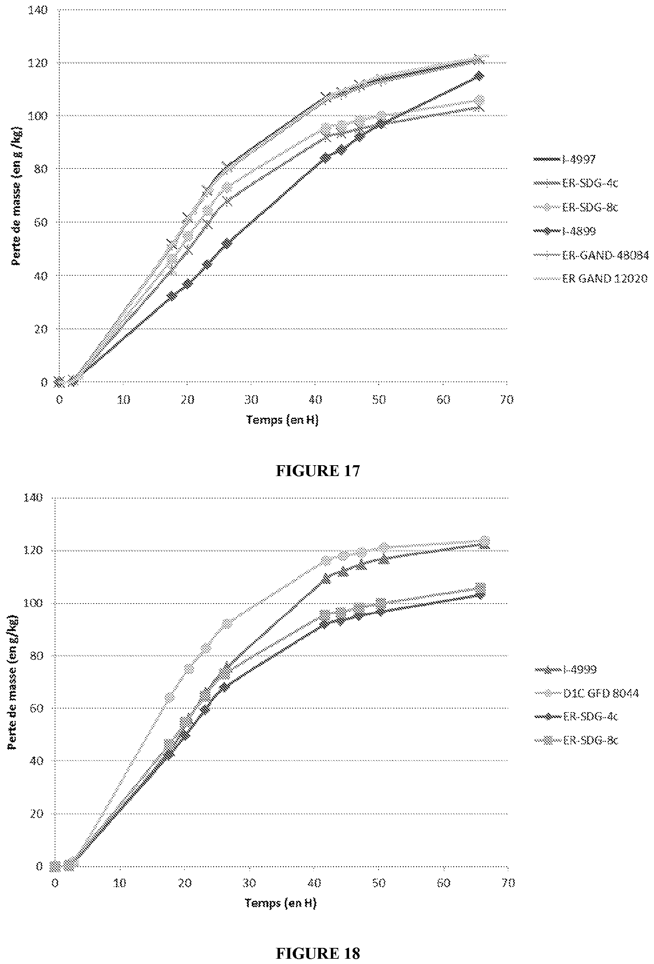

**La** **figure 1** illustre deux exemples de vecteur pANG et pSDG de surexpression et de clonage pour les glucoamylases. Ce vecteur est un vecteur de clonage intégratif utilisé pour l'expression de gènes chez la levure. *pADH1* : promoteur *d'ADH1* de *S. cerevisiae ; tCYC1 :* terminateur *CYC1* de *S. cerevisiae* ; Kan-MX : marqueur de résistance à la généticine ; AmpR : marqueur de résistance à l'ampicilline. BUD5-A et BUD5-B les régions recombinogéniques pour l'intégration au locus *BUD5.*
**La** **figure** 2 décrit la stratégie de clonage pour l'insertion de 4 modules d'expression et un module de sélection au locus *HO.*
**La** **figure 3** décrit la stratégie et les différentes étapes pour l'obtention de la souche I-5005 (A).
**La** **figure 4** illustre un exemple de résultat de criblage de 88 clones ER-GAND sur un milieu YEG/amidon. Après 48h d'incubation, l'hydrolyse de l'amidon apparait comme des halos clairs autour des colonies de levure sécrétant des glucoamylases fonctionnelles. Les levures 1 à 6 sur la 12^{ème} colonne sont des souches témoins permettant une comparaison de la taille et de l'intensité des halos.
**La** **figure 5** illustre le criblage réalisé avec les clones ER-GAND série 8000 en fermentation sur milieu dextrine. Trois durées de fermentations (20h, 31h et 54h) sont présentées. Les flèches indiquent les 15 clones ER-GAND-série 8000 sélectionnés.
**La** **figure 6** représente une fermentation sur milieu dextrine des 30 meilleurs clones ER-GAND criblés ainsi que de 4 souches témoins (ER, I-4998; I-4899 et I-4999). La fermentation est réalisée à 32°C et est suivie par perte de masse (g/kg) pendant 74h.
**La** **figure 7** représente une fermentation sur milieu industriel de maïs des 5 meilleurs clones ER-GAND série 7000 criblés ainsi que de 3 souches témoins (I-4998; I-4899 et I-4999). La fermentation est réalisée à 32°C et est suivie par une perte de masse (g/kg) pendant 70h.
**La** **figure 8** décrit la stratégie et les différentes étapes pour l'obtention des souches I-5119 (A) et I-5120 (B).
**La** **figure 9** décrit la stratégie et les différentes étapes pour l'obtention des souches témoins ER-SDG-4c et ER-SDG-8c.
**La** **figure 10** décrit la stratégie et les différentes étapes pour l'obtention de la souche ER-ANG-8c.
**La** **figure 11** représente une fermentation sur milieu dextrine des 3 meilleurs clones ER-GAND-12000 criblés ainsi que de 6 souches témoins (I-4071, I-4899, I-4997, I-4998, ER-ANG-8c, et ER-SDG-4c). La fermentation est réalisée à 32°C et est suivie par perte de masse (g/kg) pendant 70h.
**La** **figure 12** représente une fermentation sur milieu dextrine des 3 meilleurs clones ER-GAND-48000 criblés ainsi que de 4 souches témoins (I-4071, I-4899, I-4997 et ER-SDG-8c).
La fermentation est réalisée à 32°C et est suivie par perte de masse (g/kg) pendant 70h.
**La** **figure 13** illustre un exemple de vecteur pSFG de surexpression et de clonage pour les glucoamylases. Ce vecteur est un vecteur de clonage intégratif utilisé pour l'expression de gènes chez la levure. *pADH1* : promoteur *d'ADH1* de *S. cerevisiae ; tCYC1* : terminateur *CYC1* de *S. cerevisiae ; Kan-MX* : marqueur de résistance à la généticine ; AmpR : marqueur de résistance à l'ampicilline. BUD5-A et BUD5-B les régions recombinogéniques pour l'intégration au locus *BUD5.*
**La** **figure 14** décrit la stratégie et les différentes étapes pour l'obtention des souches I-5121 (A) et I-5122 (B).
**La** **figure 15** représente une fermentation sur milieu dextrine des 4 meilleurs clones ER-GFD-8000 criblés ainsi que de 3 souches témoins (I-4071, I-4999 et ER-SDG-4c). La fermentation est réalisée à 32°C et est suivie par perte de masse (g/kg) pendant 70h.
**La** **figure 16** représente une fermentation sur milieu dextrine des 4 meilleurs clones ER-GFD-48000 criblés ainsi que de 5 souches témoins (I-4071, I-4999, ER-SDG-4c, ER-SDG-8c et ER-GFD-8044). La fermentation est réalisée à 32°C et est suivie par perte de masse (g/kg) pendant 70h.
**La** **figure 17** représente une fermentation sur milieu industriel de maïs des meilleurs clones ER-GAND criblés ainsi que de 4 souches témoins (I4899, I-4997, ER-SDG-4c et ER-SDG-8c). La fermentation est réalisée à 32°C et est suivie par une perte de masse (g/kg) pendant 70h.
**La** **figure 18** représente une fermentation sur milieu industriel de maïs du meilleur clone ER-GFD série 8000 criblés ainsi que de 3 souches témoins (I-4999, ER-SDG-4c et ER-SDG-8c). La fermentation est réalisée à 32°C et est suivie par une perte de masse (g/kg) pendant 70h.

### EXEMPLES

### Exemple 1 : Intégration de 4 copies du gène codant la glucoamylase d'Aspergillus niger et d'au moins 3 copies du gène codant la glucoamylase de Saccharomyces cerevisiae var. diastaticus dans une souche de levure de Saccharomyces cerevisiae

Les copies des gènes de la glucoamylase *d'Aspergillus niger GLAA* (SEQ ID NO : 1) et de la glucoamylase de *S. cerevisiae* var. *diastaticus STA1* (SEQ ID NO : 3) ont été synthétisés au biais de codon pour *Saccharomyces cerevisiae.*

Les séquences d'ADN utilisées ont été clonées dans un vecteur-type comprenant :
- les cibles d'intégration
- les promoteurs/terminateurs choisis, par exemple *pADH1*/*tCYC1*
- les marqueurs de résistance qui pourront être éliminés par la suite.

Dans le présent exemple le plasmide pANG (dénomination interne au demandeur) a été utilisé pour exprimer la glucoamylase *GLAA d'Aspergillus niger* (cf. figure 1). De la même manière, on réalise le plasmide pSDG (dénomination interne au demandeur) pour exprimer la glucoamylase STA1 de *S. cerevisiae* var. *diastaticus*

Le principe du clonage de 4 copies de *GLAA* ou d'au moins 3 copies *STA1* peut être détaillé de la façon suivante :
- un module d'expression comprenant le promoteur *pADH1,* l'ORF des glucoamylases et le terminateur *tCYC1* a été amplifié avec 3 ou 4 couples d'oligonucléotides différents. Chaque module obtenu après amplification PCR a en commun ces 3 éléments
- Un module de sélection comprenant un promoteur/terminateur fort, un gène dont l'expression confère aux levures qui le contiennent une caractéristique permettant de les sélectionner. Il s'agit par exemple d'un gène de résistance aux antibiotiques ou d'un gène permettant la croissance de la levure sur un milieu particulier. Le module de résistance au marqueur antibiotique étant flanquée de sites LoxP, il sera possible de l'éliminer a posteriori par action de la recombinase Cre.

« ORF » a pour signification « Open Reading Frame » signifiant cadre ouvert de lecture.

Les amorces utilisées pour l'intégration des 4 copies du gène *GLAA* et du module de sélection au locus *HO* sont les suivantes :
*1ƒ-Gibson AMG :*
   TCTGATGGCTAACGGTGAAATTAAAGACATCGCAAACGTCACGGCTAACTTGAAGCTTCGTACGCTGCAGG (SEQ ID NO : 5)
*A1-Gibson AMG :*
   TCACTGTACGGTGAGAACGTAGATGGTGTGCGCATAGGCCACTAGTGGATCT (SEQ ID NO : 6)
*A2-Gibson AMG :*
   CACACCATCTACGTTCTCACCGTACAGTGAGCATAACCGCTAGAGTACTT (SEQ ID NO: 7)
*B1-Gibson AMG:*
   TTACGTAGACTGAGTAGCAACGGTTGAGGACAGCTTGCAAATTAAAGCCT (SEQ ID NO : 8)
*B2-Gibson AMG:*
   TCCTCAACCGTTGCTACTCAGTCTACGTAAGCATAACCGCTAGAGTACTT (SEQ ID NO : 9)
*C1-Gibson AMG:*
   TCAGTAGCACAGAGAAGTGTAGGAGTGTAGCAGCTTGCAAATTAAAGCCT (SEQ ID NO : 10)
*C2-Gibson AMG :*
   CTACACTCCTACACTTCTCTGTGCTACTGAGCATAACCGCTAGAGTACTT (SEQ ID NO : 11)
*D1-Gibson AMG :*
   TTAGGATACATGCAGTAGACGAGGTAAGCACAGCTTGCAAATTAAAGCCT (SEQ ID NO : 12)
*D2-Gibson AMG :*
   TGCTTACCTCGTCTACTGCATGTATCCTAAGCATAACCGCTAGAGTACTT (SEQ ID NO : 13)
*2r-Gibson AMG :*
   ACATACTTGCAATTTATACAGTGATGACCGCTGAATTTGTATCTTCCATACAGCTTGCAAATTAAAGCCT (SEQ ID NO : 14)

Les amorces utilisées pour l'intégration d'au moins 3 copies du gène *STA1* et du module de sélection au locus *GRE3* sont les suivantes :
*MCI-pADH1-GRE3-ƒ:*
   TAAGGGATATAGAAGCAAATAGTTGTCAGTGCAATCCTTCAAGACGATTGGCATAACCGCTAGAGTACTT (SEQ ID NO : 15)
*A1-tCYC1-r:*
   TCACTGTACGGTGAGAACGTAGATGGTGTGCAGCTTGCAAATTAAAGCCT (SEQ ID NO : 21)
*A2-Gibson AMG :*
   CACACCATCTACGTTCTCACCGTACAGTGAGCATAACCGCTAGAGTACTT (SEQ ID NO: 7)
*B1-Gibson AMG:*
   TTACGTAGACTGAGTAGCAACGGTTGAGGACAGCTTGCAAATTAAAGCCT (SEQ ID NO : 8)
*B2-Gibson AMG:*
   TCCTCAACCGTTGCTACTCAGTCTACGTAAGCATAACCGCTAGAGTACTT (SEQ ID NO : 9)
*C1-Gibson AMG:*
   TCAGTAGCACAGAGAAGTGTAGGAGTGTAGCAGCTTGCAAATTAAAGCCT (SEQ ID NO : 10)
*C2-Gibson AMG :*
   CTACACTCCTACACTTCTCTGTGCTACTGAGCATAACCGCTAGAGTACTT (SEQ ID NO : 11)
*D1-Gibson AMG :*
   TTAGGATACATGCAGTAGACGAGGTAAGCACAGCTTGCAAATTAAAGCCT (SEQ ID NO : 12)
*D2-Gibson AMG :*
   TGCTTACCTCGTCTACTGCATGTATCCTAAGCATAACCGCTAGAGTACTT (SEQ ID NO : 13)
*MCl-tCYC1-GRE3-r :*
   CACATATACAGCATCGGAATGAGGGAAATTTGTTCATATCGTCGTTGAGTCAGCTTGCAAATTAAAGCCT (SEQ ID NO : 16)

Le tableau 1 mentionne les couples d'oligonucléotides utilisés dans les modules de sélection et d'expression.

**Tableau 1: Couples d'amorces utilisés pour le clonage de 4 copies de GLAA et par exemple 3 ou 4 copies de STA1**

| | Couples d'amorces du module de sélection | Couples d'amorces du module d'expression pour le clonage de 4 copies de *GLAA* et 4 copies de *STA1* | Couples d'amorces du module d'expression pour le clonage de 4 copies de *GLAA* et 3 copies de *STA1* |
|---|---|---|---|
| Amorces pour le clonage du gène *ANG* | *1ƒ-Gibson AMG* + *A1-Gibson AMG* | *A2-Gibson AMG* + *B1 -Gibson AMG* | *A2-Gibson AMG* + *B1 -Gibson AMG* |
| | | *B2-Gibson AMG* + *C1-Gibson AMG* | *B2-Gibson AMG* + *C1-Gibson AMG* |
| | | *C2-Gibson AMG* + *D1-Gibson AMG* | *C2-Gibson AMG* + *D1-Gibson AMG* |
| | | *D2-Gibson AMG* + *2r-Gibson AMG* | *D2-Gibson AMG* + *2r-Gibson AMG* |
| Amorces pour le clonage du gène *SDG* | *MCI-pADH1-GRE3-f + A1-tCYC1-r* | *A2-Gibson AMG* + *B1 -Gibson AMG* | *A2-Gibson AMG* + *B1 -Gibson AMG* |
| | | *B2-Gibson AMG* + *C1-Gibson AMG* | *B2-Gibson AMG* + *C1-Gibson AMG* |
| | | *C2-Gibson AMG* + *D1-Gibson AMG* | *C2-Gibson AMG* + *MCI-tCYC1-GRE3-r* |
| | | *D2-Gibson AMG* + *MCI tCYC1-GRE3-r* | |

| | | | |
|---|---|---|---|
| « Gène *ANG* » signifie gène *GLAA* de la glucoamylase *d'Aspergillus niger.* « Gène *SDG* » signifie gène *STA1* de la glucoamylase de *Saccharomyces cerevisiae* var. *diastaticus* | | | |

Chaque module amplifié possède des séquences recombinogéniques (A1, B1, C1 et D1) de part et d'autre de son promoteur et de son terminateur. Ces séquences sont apportées par les queues flottantes des amorces PCR et permettront aux modules de s'aligner et recombiner spécifiquement par homologie entre ces séquences recombinogéniques (figure 2).

La stratégie employée consiste à intégrer simultanément plusieurs modules d'expression des gènes de glucoamylase dans une souche de *S. cerevisiae* en une seule étape à un locus donné, en se basant sur la capacité naturelle de la levure à réaliser la recombinaison homologue *in vivo.*

En fonction des combinaisons de produits PCR préparés, au moins trois modules d'expression de la glucoamylase et un module de sélection peuvent être transformés dans la souche *S*. *cerevisiae.*

La sélection des clones ayant intégré correctement les cassettes d'expression est faite dans un premier temps sur la base de la présence du module de sélection dans la cassette d'intégration (MCI).

La présence de séquences homologues à un locus donné, par exemple le locus *HO,* aux extrémités 5' et 3' de la cassette d'expression multi-intégrative permet l'intégration simultanée des modules d'expression et du module de sélection par recombinaison homologue à ce locus donné.

L'utilisation de différents marqueurs de sélection et de leur recyclage ainsi que l'intégration à différents locus, permet l'intégration séquentielle et itérative de plusieurs cassettes multi-intégratives.

Par exemple, la figure 3 illustre les différentes étapes pour l'obtention de la souche I-5005 telles qu'expliquées ci-dessous :
1- intégration de 4 modules d'expression de la glucoamylase d'A. *niger,* ci-après *GLAA,* et du module de sélection G418 (gène de résistance à la généticine / marqueur *KanMX)* au niveau du locus *HO* permettant alors d'obtenir la souche ER-ANG-G418 ;
2- élimination du module de sélection par l'action de la recombinase Cre permettant la sélection de la souche déposée le 15 octobre 2014 à la CNCM sous le numéro I-4899 ;
3- intégration d'une deuxième cassette composée de plusieurs modules d'expression de la glucoamylase de *S. cerevisiae* var. *diastaticus,* ci-après *STA1,* au niveau du locus *GRE3.*

Les souches exprimant conjointement les glucoamylases d'A. *niger (GLAA)* et de *S. cerevisiae* var. *diastaticus (STA1)* ont été nommées ER-GAND Selon ce modèle de construction, il est ainsi possible de construire des levures ayant intégré au moins 4 copies du gène de glucoamylase *GLAA* et au moins 3 copies du gène de glucoamylase *STA1.*

Pour les levures ER-GAND deux séries de clones ont été générées. La série 7000 (ER-GAND-7200 à ER-GAND-7376) correspond à l'intégration de 4 copies du gène de glucoamylase *GLAA (d'A. niger)* et 3 copies du gène de glucoamylase *STA1* (de *S. cerevisiae* var. *diastaticus*)*.* Quant à la série 8000 (ER-GAND-8000 à ER-GAND-8159), au moins 4 copies du gène de glucoamylase *GLAA (d'A. niger)* et 4 copies du gène de glucoamylase *STA1* (de *S. cerevisiae* var. *diastaticus*) ont été clonées. Le clone 8159 correspond à la souche I-4997.

Les souches de levure utilisées sont rappelées ci-dessous dans le tableau 2 ainsi que leurs caractéristiques.

**Tableau 2 : Récapitulatif des noms/ numéros de souches et du nombre de copies de gènes de glucoamylase intégrés (nd : non déposé)**

| *Nom souches* | *CNCM* | *Copies GA* - *ANG* | *Copies GA de SDG* | *Copies GA de SFG* |
|---|---|---|---|---|
| ER | I-4071 | 0 | 0 | 0 |
| ER-ANG-G418 | nd | 4 | 0 | 0 |
| ER-GA-36 | I-4899 | 4 | 0 | 0 |
| ER-SFG-4c | I-4999 | 0 | 0 | 4 |
| ER-SDG-3c | I-4998 | 0 | 3 | 0 |

### Exemple 2 : Criblage des souches

Trois criblages phénotypiques ont été réalisés afin de sélectionner les quinze meilleurs clones les plus performants pour l'application visée.

### a) Criblage phénotypique à l'iode

L'hydrolyse de l'amidon soluble par les transformants de levure est testée sur un milieu agar YEG/amidon (Glucose 1%, extrait de levure 0.5%, amidon soluble 1%). Les cellules de levure sont déposées sur la gélose YEG/amidon et incubées pendant 2 jours à 30°C. Ensuite les boites sont colorées à la vapeur d'iode afin de visualiser les halos d'hydrolyse présents autour des colonies de levure.

Ce criblage à la vapeur d'iode permet de sélectionner les clones sécrétant au moins une enzyme capable d'hydrolyser l'amidon. Ces clones positifs sont visualisables notamment par des halos d'hydrolyse dont la taille est proportionnelle à l'activité enzymatique. La figure 4 illustre un exemple de criblage de 88 clones ER-GAND sur milieu YEG/amidon après coloration à l'iode. Le tableau 3 présente les résultats obtenus après coloration à l'iode.

**Tableau 3 : Résultats du criblage à l'iode pour les 2 séries ER-GAND**

| ***Souches ER-GAND*** | ***Nombre de clones criblés*** | ***Croissance sur YEG*** | ***Aucun phénotype d'hydrolyse*** | ***Phénotype** d'hydrolyse **plus faible que le témoin*** |
|---|---|---|---|---|
| Série 7000 | 176 | 173 (98%) | 1 (<1%) | 0 (0%) |
| Série 8000 | 176 | 176 (100%) | 5 (3%) | 1 (<1%) |

Sur 176 clones criblés pour chaque série, moins de 3% des clones testés ne semblent pas être capables d'hydrolyser l'amidon dans les conditions de culture de l'exemple. A noter que la souche hôte utilisée dans cette stratégie possédait déjà plusieurs gènes de glucoamylase dans son génome, ainsi un halo d'hydrolyse est présent pour cette souche. Ces clones « négatifs » semblent donc avoir perdu leurs gènes de glucoamylase.

### b) Criblage phénotypique en fermentation dans un milieu de 0.5 g.

Le criblage phénotypique sur un milieu dextrine en condition de fermentation permet d'éliminer les clones ER-GAND ne pouvant pas fermenter ou fermentant plus lentement que la souche I-4899. Pour cela, un suivi visuel de la biomasse au cours de la fermentation est réalisé deux fois par jour pendant 3 jours. En comparant la vitesse d'apparition du culot de biomasse par rapport aux souches témoins I-4999 et I-4998, les souches les plus prometteuses peuvent alors être sélectionnées.

La souche ER-SDG-1c mentionnée dans les témoins de la figure 5 est une souche Ethanol Red ® exprimant une copie du gène de la glucoamylase de *S. cerevisiae* var. *diastaticus (STA1).*

La souche GO-ANG-4c mentionnée dans les témoins de la figure 5 est une souche GenOne+® déposée à la CNCM le 25 juillet 2013 sous le numéro I-4791 exprimant quatre copies du gène de la glucoamylase d'A. *niger (GLAA)*

Le milieu de fermentation utilisé, le milieu dextrine, est un milieu synthétique contenant des dextrines d'amidon (220 g/kg), de l'extrait de levure (5 g/kg), de l'urée (2 g/kg), du KH₂PO₄ (1 g/kg) ainsi que des minéraux et des vitamines. Les souches fermentant le plus vite sont des souches capables de sécréter une quantité importante de glucoamylase permettant alors de libérer du glucose par l'hydrolyse des molécules de dextrine. Le glucose ainsi libéré est alors métabolisé par la levure *S. cerevisiae* afin de produire de l'éthanol.

La figure 5 illustre un exemple de fermentation en plaque pour les 88 clones ER-GAND. Les culots de biomasse correspondent à une fermentation de 31 h sur milieu dextrine. Les clones indiqués par une flèche présentent un culot de biomasse plus important que pour la souche I-4899. Ce sont ces clones qui ont été sélectionnés pour être testés lors d'une fermentation sur 100 g de milieu dextrine.

Pour chaque série ER-GAND, quinze clones ont été sélectionnés et vont être testés lors d'une fermentation sur 100 g de milieu dextrine. c) Criblage phénotypique en fermentation dans un milieu de 100 g.

Une fermentation sur 100 g de milieu synthétique sélectif dextrine (dextrine de maïs 220 g/kg, extrait de levure 5 g/kg, urée 2 g/kg, KH₂PO₄ 1 g/kg ainsi que des minéraux et des vitamines) est réalisée à 32°C. Les dextrines sont des hydrolysats d'amidon permettant de mimer le milieu réel.

Les souches de *S. cerevisiae* modifiées selon l'invention ont été préalablement propagées sur un milieu YPG (Yeast extract, Peptone, Glucose) pendant 24h à 30°C. Le pH initial du milieu de fermentation a été ajusté à 5.0 sans régulation. Le milieu de fermentation a ensuite été inoculé à un taux de 0.125 g équivalent matière sèche par kilogramme de milieu. Aucune enzyme d'hydrolyse exogène n'est ajoutée au milieu de fermentation. Un suivi de perte de masse a été réalisé pendant 72 heures et est illustré figure 6.

Dans ce type de milieu de fermentation (dextrine), il y a peu de glucose libre à t0 (environ une dizaine de grammes). La souche ER, n'ayant pas d'enzyme pouvant hydrolyser les dextrines, elle consomme uniquement le glucose disponible et donc une faible perte de masse est mesurée (environ 5 g/kg).

A partir des résultats de suivi de perte de masse présentés figure 6, sur 30 clones testés 3 groupes peuvent être établis selon leurs comportements cinétiques en fermentation:
- Groupe A : 4 clones ont un profil cinétique identique à la souche de levure mère I-4899.
- Groupe B : 2 clones présentent un profil cinétique similaire à la souche de référence I-4999
- Groupe C : la performance en cinétique de fermentation de 24 clones est supérieure à celle de la souche I-4999 (performance cible)

Parmi les souches du groupe C, les 5 meilleurs clones de chaque série (séries 7000 et 8000) ont été sélectionnés pour être testés sur un milieu industriel en condition réel de production de biocarburant.

### Exemple 3 : Evaluation de la production d'activité enzymatique des souches

Les 5 clones ER-GAND avec 4 copies de *GLAA* et 3 copies de *STA1* (série 7 000) sélectionnés précédemment, ont été évaluées à 32°C sur le milieu industriel E140723-11 et comparées avec les souches témoin I-4998, I-4999 et I-4899. Il s'agit des clones suivants : 7215, 7250, 7271, 7296, 7302. Le clone 7302 correspond à la souche I-5005.

La souche (déposée le 9 juillet 2015 à la CNCM sous le numéro I-4998) exprimant l'activité *STA1* issue de *S. cerevisiae* var. *diastaticus* permettait d'obtenir une cinétique d'hydrolyse des dextrines rapide mais incomplète, alors que la souche (I-4899) exprimant l'activité *GLAA* issue *d'A. niger* permettait d'obtenir une hydrolyse des dextrines satisfaisante mais avec une cinétique inférieure à I-4998.

Les souches ont été préalablement propagées sur un mélange milieu/eau (70%/30%) pendant 7h30 à 32°C. Le milieu de propagation a ensuite été transféré vers le milieu de fermentation à un taux de 2.5%/97.5%. La fermentation a été réalisée à 32°C. Le pH initial des milieux de propagation et de fermentation ont été ajustés à 5.0 sans régulation. De l'urée a été ajoutée en propagation (1500 ppm) et en fermentation (1000 ppm). Une dose de 0.06 mL/kg de solides de glucoamylase GA commerciale Spirizyme® Ultra (Novozyme) a été ajoutée en propagation mais pas en fermentation.

On a mesuré au cours du temps de t=0 à t=71 h la perte de masse des réacteurs de fermentation.

Les résultats de pertes de masse obtenues au cours de la fermentation alcoolique sont présentés sur la Figure 7.

La figure 7 montre que les nouvelles souches permettent d'obtenir à la fois une hydrolyse des dextrines aussi rapide que la souche I-4998 et complète comme la souche I-4899, mais qu'elles sont également plus rapides que la souche I-4999. On peut en conclure que la production de glucoamylase de *Saccharomyces cerevisiae* var. *diastaticus* a un effet stimulant sur l'activité hydrolytique de la glucoamylase *Aspergillus niger.* Ces souches ne font pas que de présenter une combinaison des caractéristiques de la glucoamylase *d'Aspergillus niger* fongique et de celle de *Saccharomyces cerevisiae* var. *diastaticus* (c'est-à-dire avec un profil cinétique qui se situerait entre celui de la glucoamylase *d'Aspergillus niger* et celui de la glucoamylase de *Saccharomyces cerevisiae* var. *diastaticus),* mais présentent un profil cinétique amélioré avec une accélération de la cinétique. Il y aurait donc un effet synergique entre la glucoamylase d'origine fongique et celle de *Saccharomyces cerevisiae* var. *diastaticus*

La composition des échantillons de fermentation est mesurée par chromatographie en phase liquide à haute performance (HPLC) sur une colonne Aminex® HPX 87H (Biorad) avec une solution d'H₂SO₄ 5 mM comme éluant.

Les HPLC réalisées en fin de fermentation ne montrent pas de défaut majeur pour aucune des souches considérées (tableau 4). Elles rappellent bien l'incapacité de l'enzyme STA1 d'hydrolyser totalement les dextrines contrairement aux enzymes SFG et GLAA.

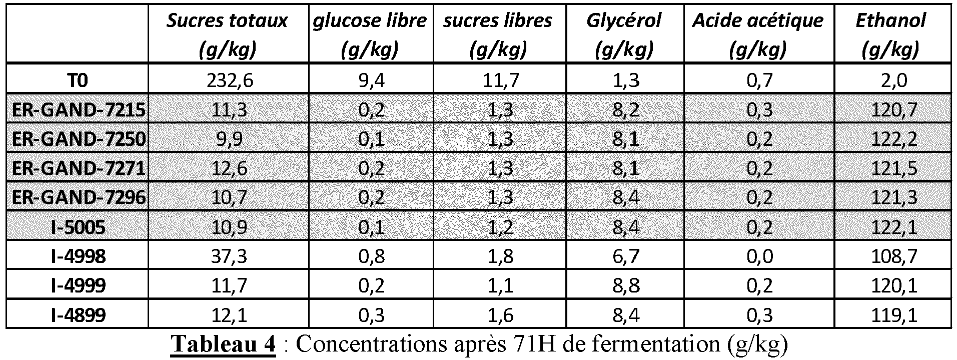

### Exemple 4 : Intégration de 4 copies supplémentaires d'un gène codant pour une glucoamylase dans une souche de levure de Saccharomyces cerevisiae possédant au moins 4 copies du gène codant la glucoamylase d'Aspergillus niger et d'au moins 4 copies du gène codant la glucoamylase de Saccharomyces cerevisiae var. diastaticus

A partir du clone ER-GAND-8159 (CNCM I-4997), des copies supplémentaires du gène d'une glucoamylase ont été intégrées dans le locus *BUD5* afin d'augmenter le nombre de copie du gène d'une des deux glucoamylases.

Dans le présent exemple, les séquences utilisées pour cette intégration correspondent aux gènes de la glucoamylase *d'Aspergillus niger GLAA* (SEQ ID NO : 1) et de la glucoamylase de *S. cerevisiae* var. *diastaticus STA1* (SEQ ID NO : 3) précédemment décrites.

Le principe général du clonage est le même que celui décrit dans l'exemple 1, seul le locus d'intégration varie.

Le principe du clonage de 4 copies supplémentaires de *GLAA* ou de 4 copies supplémentaires de *STA1* peut être détaillé de la façon suivante :
- un module d'expression comprenant le promoteur *pADH1,* l'ORF des glucoamylases et le terminateur *tCYC1* a été amplifié avec 4 couples d'oligonucléotides différents. Chaque module obtenu après amplification PCR a en commun ces 3 éléments
- Un module de sélection comprenant un promoteur/terminateur fort, un gène dont l'expression confère aux levures qui le contiennent une caractéristique permettant de les sélectionner. Il s'agit par exemple d'un gène de résistance aux antibiotiques ou d'un gène permettant la croissance de la levure sur un milieu particulier. Le module de résistance au marqueur antibiotique étant flanquée de sites LoxP, il sera possible de l'éliminer a posteriori par action de la recombinase Cre.

Egalement, des souches de levure de *Saccharomyces cerevisiae* exprimant exclusivement soit la glucoamylase d'A. *niger* soit la glucoamylase de *S. cerevisiae* var. *diastaticus* ont été obtenues selon la même stratégie de clonage. Ces souches de levure de *S. cerevisiae* peuvent alors contenir 4 ou 8 copies du gène de la même glucoamylase au sein d'un ou des deux locus.

Les amorces utilisées pour l'intégration des différentes copies du gène *GLAA* ou du gène *STA1* et du module de sélection sont les suivantes :
*MCI-pADH1-BUD5-ƒ:*
   CGCTCCAGAATTAGCGGACCTCTTGAGCGGTGAGCCTCTGGCAAAGAAGAGCATAACCGCTAGAGTACTT (SEQ ID NO : 19)
*MCI-pTEF-BUD5-ƒ:*
   CGCTCCAGAATTAGCGGACCTCTTGAGCGGTGAGCCTCTGGCAAAGAAGATGAAGCTTCGTACGCTGCAGG (SEQ ID NO : 20)
*MCI-pADH1-GRE3-ƒ:*
   TAAGGGATATAGAAGCAAATAGTTGTCAGTGCAATCCTTCAAGACGATTGGCATAACCGCTAGAGTACTT (SEQ ID NO : 15)
*A1-tCYC1-r:*
   TCACTGTACGGTGAGAACGTAGATGGTGTGCAGCTTGCAAATTAAAGCCT (SEQ ID NO : 21)
*A2-Gibson AMG :*
   CACACCATCTACGTTCTCACCGTACAGTGAGCATAACCGCTAGAGTACTT (SEQ ID NO : 7)
*B1-Gibson AMG:*
   TTACGTAGACTGAGTAGCAACGGTTGAGGACAGCTTGCAAATTAAAGCCT (SEQ ID NO : 8)
*B2-Gibson AMG:*
   TCCTCAACCGTTGCTACTCAGTCTACGTAAGCATAACCGCTAGAGTACTT (SEQ ID NO : 9)
*C1-Gibson AMG:*
   TCAGTAGCACAGAGAAGTGTAGGAGTGTAGCAGCTTGCAAATTAAAGCCT (SEQ ID NO : 10)
*C2-Gibson AMG :*
   CTACACTCCTACACTTCTCTGTGCTACTGAGCATAACCGCTAGAGTACTT (SEQ ID NO : 11)
*D1-Gibson AMG :*
   TTAGGATACATGCAGTAGACGAGGTAAGCACAGCTTGCAAATTAAAGCCT (SEQ ID NO : 12)
*D2-Gibson AMG :*
   TGCTTACCTCGTCTACTGCATGTATCCTAAGCATAACCGCTAGAGTACTT (SEQ ID NO : 13)
*MCl-tCYC1-BUD5-r :*
   CTCAAGAACGTAGGACGATAACTGGTTGGAAAGCGTAAACACGGAGTCAACAGCTTGCAAATTAAAGCCT (SEQ ID NO : 22)
*MCl-tCYC1-GRE3-r :*
   CACATATACAGCATCGGAATGAGGGAAATTTGTTCATATCGTCGTTGAGTCAGCTTGCAAATTAAAGCCT (SEQ ID NO : 16)

Le tableau 5 mentionne les couples d'oligonucléotides utilisés dans les modules de sélection et d'expression, ainsi que la souche de levure hôte pour les différentes constructions.

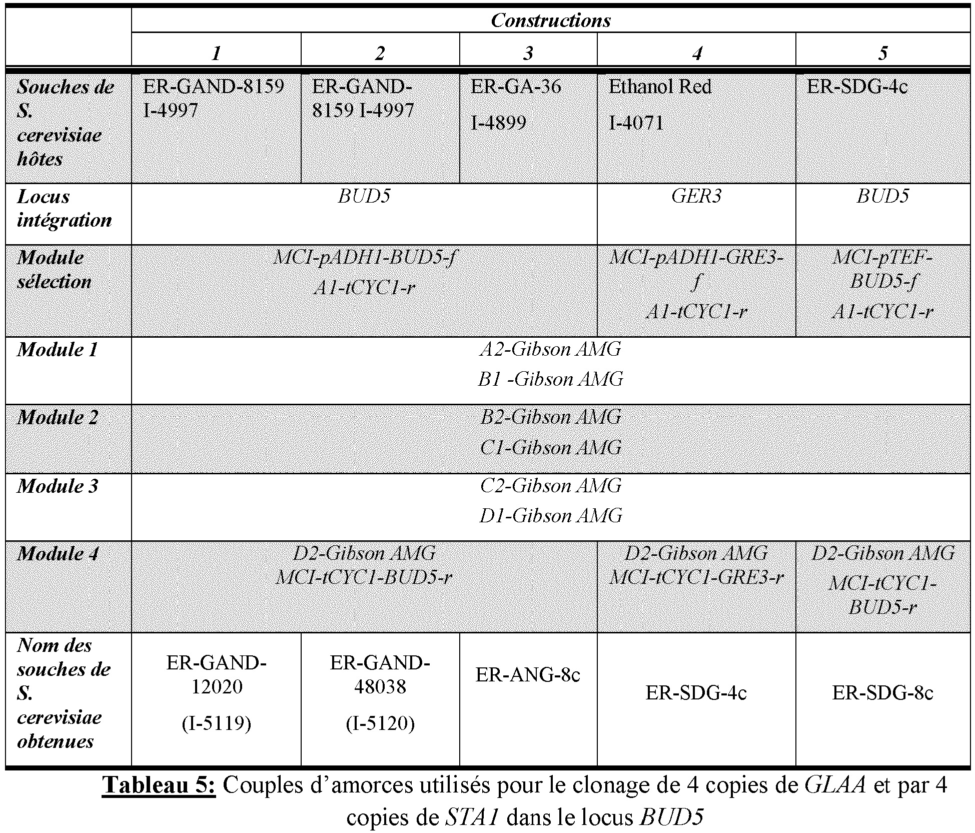

La stratégie est strictement similaire à celle employée dans l'exemple 1 pour l'intégration simultanée de plusieurs modules d'expression des gènes de glucoamylase dans une souche de *S. cerevisiae* en une seule étape à un locus donné, en se basant sur la capacité naturelle de la levure à réaliser la recombinaison homologue *in vivo.*

Les souches exprimant conjointement les glucoamylases d'A. *niger (GLAA)* et de *S. cerevisiae* var. *diastaticus (STA1)* ont été nommées ER-GAND plus le numéro du clone. Deux séries de clones ont été générées. La série 12000 (ER-GAND-12001 à 12023) correspond à l'intégration de 8 copies du gène de glucoamylase *GLAA (d'A. niger)* et 4 copies du gène de glucoamylase *STA1* (de *S. cerevisiae* var. *diastaticus*) et la série 48000 (ER-GAND-480001 à 480088) correspond à l'intégration de 4 copies du gène de glucoamylase *GLAA* (*d'A. niger*) et de 8 copies du gène de glucoamylase *STA1* (de *S. cerevisiae* var. *diastaticus).*

Les souches exprimant exclusivement la glucoamylase d'A. *niger (GLAA)* ont été nommées ER-ANG-z-c où z correspond au nombre de copie du gène *GLAA* introduit dans la souche hôte. Les souches exprimant exclusivement la glucoamylase *S. cerevisiae* var. *diastaticus (STA1)* ont été nommées ER-SDG-y-c où y correspond au nombre de copie du gène *STA1* introduit dans la souche hôte.

Par exemple, les figures 8 à 10 illustrent les différentes étapes pour l'obtention des souches ER-GAND-12000, ER-GAND-48000, ER-ANG-8c, ER-SDG-4c, ER-SDG-8c construites dans cet exemple.

Les souches de levure utilisées sont rappelées ci-dessous dans le tableau 6 ainsi que leurs caractéristiques.

**Tableau 6 : Récapitulatif des noms/ numéros de souches et du nombre de copies de gènes de glucoamylase intégrés (nd : non déposé)**

| *Nom souches* | *CNCM* | *Copies GA -ANG* | *Copies GA de SDG* |
|---|---|---|---|
| ER | I-4071 | 0 | 0 |
| ER-GA-36 | I-4899 | 4 | 0 |
| ER-SDG-4c | nd | 0 | 4 |
| ER-SDG-8c | nd | 0 | 8 |
| ER-ANG-8c | nd | 8 | 4 |
| ER-GAND-12020 | I-5119 | 8 | 4 |
| ER-GAND-48035 | I-5120 | 4 | 8 |

### Exemple 5 : Criblage des souches

Trois criblages phénotypiques ont été réalisés afin de sélectionner les meilleurs clones les plus performants pour l'application visée.

### a) Criblage phénotypique à l'iode

Le criblage phénotypique à l'iode a été effectué de manière identique à l'Exemple 2a).. Le tableau 7 présente les résultats obtenus après coloration à l'iode.

**Tableau 7 : Résultats du criblage à l'iode pour les 5 séries de clonage réalisé. Le phénotype d'hydrolyse plus faible correspond à un halo autour du clone plus petit que celui de la souche de S. cerevisiae hôte**

| ***Souches de S. cerevisiae*** | ***Nombre de clones criblés*** | ***Croissance sur YEG*** | ***Aucun phénotype d'hydrolyse*** | ***Phénotype d'hydrolyse plus faible^{*}*** |
|---|---|---|---|---|
| *ER-ANG-8c* | 88 | 0 | 0 | 0 |
| *ER-SDG-4c* | 132 | 0 | 3 (2%) | - |
| *ER-SDG-8c* | 9 | 0 | 0 | 0 |
| *ER-GAND série 12 000* | 23 | 0 | 0 | 1 (4%) |
| *ER-GAND série 48 000* | 88 | 0 | 0 | 0 |

Sur l'ensemble des 5 séries de criblage réalisé, seule la série pour ER-SDG-4c présente quelques clones n'ayant pas d'activité d'hydrolyse de l'amidon dans les conditions de culture de l'exemple. Il est à noter que c'est la seule série où le clonage a été réalisé dans la souche hôte Ethanol Red® qui ne possède pas de gène de glucoamylase. Ces clones « négatifs » semblent donc n'avoir pas intégré au moins un gène de glucoamylase.

### b) Criblage phénotypique en fermentation dans un milieu de 0.5 g.

Le criblage phénotypique sur un milieu dextrine en condition de fermentation permet d'éliminer les clones obtenus dans les différentes séries qui ne peuvent pas fermenter ou fermentant plus lentement que la souche hôte correspondante. Pour cela, un suivi visuel de la biomasse au cours de la fermentation est réalisé deux fois par jour pendant 2 jours. En comparant la vitesse d'apparition du culot de biomasse par rapport à la souche témoin I-4997, les souches les plus prometteuses peuvent alors être sélectionnées.

Le milieu de fermentation utilisé, le milieu dextrine, est un milieu synthétique contenant des dextrines d'amidon (220 g/kg), de l'extrait de levure (5 g/kg), de l'urée (2 g/kg), du KH₂PO₄ (1 g/kg) ainsi que des minéraux et des vitamines. Les souches fermentant le plus vite sont des souches capables de sécréter une quantité importante de glucoamylase permettant alors de libérer du glucose par l'hydrolyse des molécules de dextrine. Le glucose ainsi libéré est alors métabolisé par la levure *S. cerevisiae* afin de produire de l'éthanol.

Pour chaque série de clonage, entre deux et quatre clones ont été sélectionnés et sont testés à une échelle plus grande lors d'une fermentation sur 100 g de milieu dextrine.

### c) Criblage phénotypique en fermentation dans un milieu de 100 g.

Comme pour l'exemple 2c), une fermentation sur 100 g de milieu synthétique sélectif dextrine (dextrine de maïs 220 g/kg, extrait de levure 5 g/kg, urée 2 g/kg, KH₂PO₄ 1 g/kg ainsi que des minéraux et des vitamines) est réalisée à 32°C. Les dextrines sont des hydrolysats d'amidon permettant de mimer le milieu réel.

Les souches de *S. cerevisiae* modifiées selon l'invention ont été préalablement propagées sur un milieu YPG (Yeast extract, Peptone, Glucose) pendant 24h à 30°C. Le pH initial du milieu de fermentation a été ajusté à 5.0 sans régulation. Le milieu de fermentation a ensuite été inoculé à un taux de 0.125 g équivalent matière sèche par kilogramme de milieu. Aucune enzyme d'hydrolyse exogène n'est ajoutée au milieu de fermentation. Des suivis de perte de masse ont été réalisés pendant 72 heures et sont illustrés figure 11, pour la série ER-GAND-12000 et figure 12 pour la série ER-GAND-48000.

Dans ce type de milieu de fermentation (dextrine), il y a peu de glucose libre à t0 (environ une dizaine de grammes). La souche ER, n'ayant pas d'enzyme pouvant hydrolyser les dextrines, elle consomme uniquement le glucose disponible et donc une faible perte de masse est mesurée (environ 5 g/kg) à partir de 14h jusqu'à la fin de la fermentation.

A partir des résultats de suivi de perte de masse présentés figure 11 et 12, les 3 clones testés pour chaque série présentent des cinétiques de perte de masse quasi-identiques et semblent donc équivalent en performance fermentaire sur un milieu dextrine.

Egalement, l'augmentation du nombre de copie du gène *STA1* ou *sGLAA* (de 4 à 8 copies) dans une souche de *S. cerevisiae* permet de gagner en performance cinétique, notamment pour la vitesse de fermentation au cours des 30 premières heures (figure 11).

L'ajout également de soit 4 copies du gène *sGLAA* (figure 11) ou de 4 copies du gène de *STA1* (figure 12) dans la souche ER-GAND-8159 (I-4997), permet une amélioration de la cinétique de fermentation sur milieu dextrine et ainsi de combiner les performances des glucoamylases de *S. cerevisiae* var. *diataticus* et d'A. *niger.*

### Exemple 6 : Intégration de 4 ou 8 copies du gène codant pour la glucoamylase de Saccharomyces cerevisiae var. diastaticus dans une souche de levure de Saccharomyces cerevisiae contenant 4 copies du gène codant la glucoamylase de Saccharomycopsis fibuligera

Les copies des gènes de la glucoamylase de *S. cerevisiae* var. *diastaticus STA1* (SEQ ID NO : 3) et de la glucoamylase de *Saccharomycopsisfibuligera GLU0111* (SEQ ID NO : 17) ont été synthétisés au biais de codon pour *Saccharomyces cerevisiae.*

Les séquences d'ADN utilisées ont été clonées dans un vecteur-type comprenant :
- les cibles d'intégration
- les promoteurs/terminateurs choisis, par exemple pADH1/tCYC1
- les marqueurs de résistance qui pourront être éliminés par la suite.

Dans le présent exemple le plasmide pSFG (dénomination interne au demandeur) a été utilisé pour exprimer la glucoamylase *GLU0111* de *Saccharomycopsis fibuligera* (cf. figure 13). De la même manière, on réalise le plasmide pSDG (dénomination interne au demandeur) pour exprimer la glucoamylase *STA1* de *S. cerevisiae* var. *diastaticus*

Le principe du clonage de 4 copies de *GLU0111* ou de 4 ou 8 copies *STA1* peut être détaillé de la façon suivante :
- un module d'expression comprenant le promoteur *pADH1,* l'ORF des glucoamylases et le terminateur tCYC1 a été amplifié avec 3 ou 4 couples d'oligonucléotides différents. Chaque module obtenu après amplification PCR a en commun ces 3 éléments
- Un module de sélection comprenant un promoteur/terminateur fort, un gène dont l'expression confère aux levures qui le contiennent une caractéristique permettant de les sélectionner. Il s'agit par exemple d'un gène de résistance aux antibiotiques ou d'un gène permettant la croissance de la levure sur un milieu particulier. Le module de résistance au marqueur antibiotique étant flanquée de sites LoxP, il sera possible de l'éliminer a posteriori par action de la recombinase Cre.

Les amorces utilisées pour l'intégration des différentes copies du gène *GLAA* ou du gène *STA1* et du module de sélection sont les suivantes :
*1ƒ-Gibson AMG :*
*MCI-pTEF-BUD5-ƒ:*
*MCI-ADH1-GRE3-ƒ:*
*A1-Gibson AMG :*
   TCACTGTACGGTGAGAACGTAGATGGTGTGCGCATAGGCCACTAGTGGATCT (SEQ ID NO : 6)
*A1-tCYC1-r:*
   TCACTGTACGGTGAGAACGTAGATGGTGTGCAGCTTGCAAATTAAAGCCT (SEQ ID NO : 21)
*A2-Gibson AMG :*
   CACACCATCTACGTTCTCACCGTACAGTGAGCATAACCGCTAGAGTACTT (SEQ ID NO: 7)
*B1-Gibson AMG:*
   TTACGTAGACTGAGTAGCAACGGTTGAGGACAGCTTGCAAATTAAAGCCT (SEQ ID NO : 8)
*B2-Gibson AMG:*
   TCCTCAACCGTTGCTACTCAGTCTACGTAAGCATAACCGCTAGAGTACTT (SEQ ID NO : 9)
*C1-Gibson AMG:*
   TCAGTAGCACAGAGAAGTGTAGGAGTGTAGCAGCTTGCAAATTAAAGCCT (SEQ ID NO : 10)
*C2-Gibson AMG :*
   CTACACTCCTACACTTCTCTGTGCTACTGAGCATAACCGCTAGAGTACTT (SEQ ID NO: 11)
*D1-Gibson AMG :*
   TTAGGATACATGCAGTAGACGAGGTAAGCACAGCTTGCAAATTAAAGCCT (SEQ ID NO : 12)
*D2-Gibson AMG :*
   TGCTTACCTCGTCTACTGCATGTATCCTAAGCATAACCGCTAGAGTACTT (SEQ ID NO : 13)
*MCI-tCYC1-BUD5-r :*
*MCI-tCYC1-GRE3-r :*
*2r-Gibson AMG :*

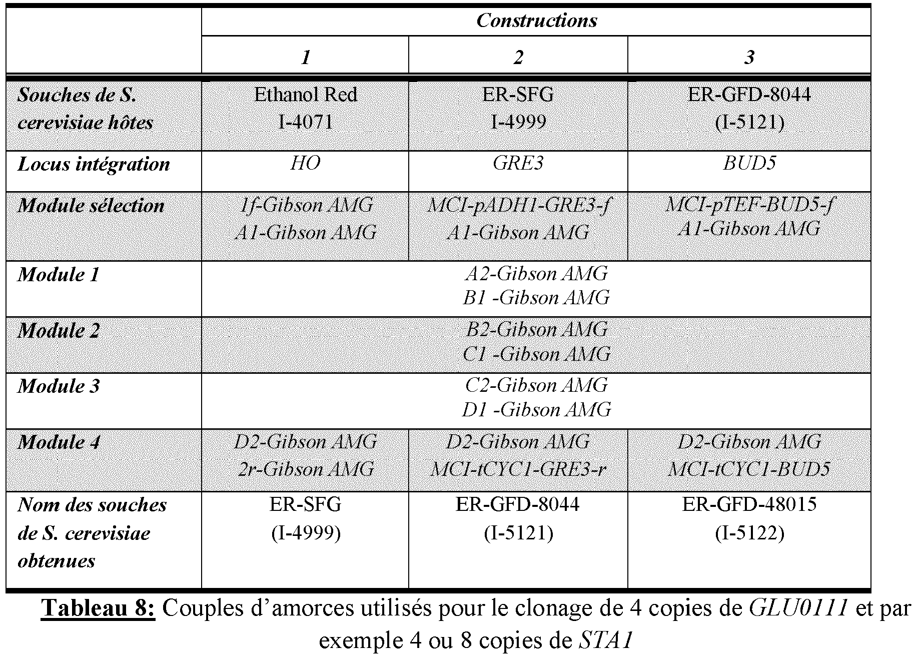

« Gène *SFG* » signifie gène *GLU0111de* la glucoamylase de *Saccharomycopsis fibuligera.*

La stratégie est strictement similaire à celle employée dans l'exemple 1 pour l'intégration simultanée de plusieurs modules d'expression des gènes de glucoamylase dans une souche de *S. cerevisiae* en une seule étape à un locus donné, en se basant sur la capacité naturelle de la levure à réaliser la recombinaison homologue *in vivo.*

Par exemple, la figure 14 illustre les différentes étapes pour l'obtention de la souche I-5122 telles qu'expliquées ci-dessous :
1- intégration de 4 modules d'expression de la glucoamylase de *S. fibuligera,* ci-après *GLU0111,* et du module de sélection G418 (gène de résistance à la généticine / marqueur *KanMX)* au niveau du locus *HO* permettant alors d'obtenir la souche ER-SFG;
2- intégration d'une deuxième cassette composée de 4 modules d'expression de la glucoamylase de *S. cerevisiae* var. *diastaticus,* ci-après *STA1,* au niveau du locus *GRE3.*
3- intégration d'une troisième cassette composée de 4 modules d'expression de la glucoamylase de *S. cerevisiae* var. *diastaticus,* ci-après *STA1,* au niveau du locus *BUD5*

Les souches exprimant conjointement les glucoamylases de *S. fibuligera (GLU0111)* et de *S. cerevisiae* var. *diastaticus (STA1)* ont été nommées ER-GFD. Selon ce modèle de construction, il est ainsi possible de construire des levures ayant intégré 4 copies du gène de glucoamylase *GLU0111* et au moins 4 copies du gène de glucoamylase *STA1.*

Pour les levures ER-GFD deux séries de clones ont été générées. La série 8000 (ER-GFD-8001 à ER-GFD-8045) correspond à l'intégration de 4 copies du gène de glucoamylase *GLU0111* (de *S. fibuligera)* et 4 copies du gène de glucoamylase *STA1* (de *S. cerevisiae* var. *diastaticus).* Quant à la série 48000 (ER-GFD-48001 à ER-GFD-48015), 4 copies du gène de glucoamylase *GLU0111* (de *S. fibuligera)* et 8 copies du gène de glucoamylase *STA1* (de *S. cerevisiae* var. *diastaticus)* ont été clonées. Les clones ER-GFD-8044 et ER-GFD-48015 correspondent aux souches I-5121 et I-5122, respectivement.

Les souches de levure utilisées sont rappelées ci-dessous dans le tableau 9 ainsi que leurs caractéristiques.

**Tableau 9 : Récapitulatif des noms/ numéros de souches et du nombre de copies de gènes de glucoamylase intégrés**

| *Nom souches* | *CNCM* | *Copies GA de SDG* | *Copies GA de SFG* |
|---|---|---|---|
| ER | I-4071 | 0 | 0 |
| ER-SFG-4c | I-4999 | 0 | 4 |
| ER-GFD-8044 | I-5121 | 4 | 4 |
| ER-GFD-48015 | I-5122 | 8 | 4 |

### Exemple 7 : Criblage des souches

Trois criblages phénotypiques ont été réalisés afin de sélectionner les meilleurs clones les plus performants pour l'application visée.

### a) Criblage phénotypique à l'iode

Le criblage phénotypique à l'iode a été effectué de manière identique à l'Exemple 2a).

Le tableau 10 présente les résultats obtenus après coloration à l'iode.

**Tableau 10 : Résultats du criblage à l'iode pour les 2 séries de clonage réalisé. Le phénotype d'hydrolyse plus faible correspond à un halo autour du clone plus petit que celui de la souche de S. cerevisiae hôte**

| ***Souches de S. cerevisiae*** | ***Nombre de clones criblés*** | ***Croissance sur YEG*** | ***Aucun phénotype d'hydrolyse*** | ***Phénotype d'hydrolyse plus faible^{*}*** |
|---|---|---|---|---|
| *ER-GFD série 8 000* | 45 | 0 | 0 | 1 (2%) |
| *ER-GFD série 48 000* | 15 | 0 | 0 | 0 |

Sur l'ensemble des clones criblés pour chaque série, moins de 2% des clones testés ne semblent pas être capables d'hydrolyser l'amidon dans les conditions de culture de l'exemple. A noter que la souche hôte utilisée dans cette stratégie possédait déjà plusieurs gènes de glucoamylase dans son génome, ainsi un halo d'hydrolyse est présent pour cette souche. Ces clones « négatifs » semblent donc avoir perdu leurs gènes de glucoamylase.

### b) Criblage phénotypique en fermentation dans un milieu de 0.5 g.

Le criblage phénotypique sur un milieu dextrine en condition de fermentation permet d'éliminer les clones obtenus dans les différentes séries qui ne peuvent pas fermenter ou fermentant plus lentement que la souche hôte correspondante. Pour cela, un suivi visuel de la biomasse au cours de la fermentation est réalisé deux fois par jour pendant 2 jours. En comparant la vitesse d'apparition du culot de biomasse par rapport à la souche témoin I-4999, les souches les plus prometteuses peuvent alors être sélectionnées.

Le milieu de fermentation utilisé, le milieu dextrine, est un milieu synthétique contenant des dextrines d'amidon (220 g/kg), de l'extrait de levure (5 g/kg), de l'urée (2 g/kg), du KH₂PO₄ (1 g/kg) ainsi que des minéraux et des vitamines. Les souches fermentant le plus vite sont des souches capables de sécréter une quantité importante de glucoamylase permettant alors de libérer du glucose par l'hydrolyse des molécules de dextrine. Le glucose ainsi libéré est alors métabolisé par la levure *S. cerevisiae* afin de produire de l'éthanol.

Pour chaque série de clonage, entre deux et quatre clones ont été sélectionnés et sont testés à une échelle plus grande lors d'une fermentation sur 100 g de milieu dextrine.

### c) Criblage phénotypique en fermentation dans un milieu de 100 g.

Comme pour les exemples 2c) et 5c), une fermentation sur 100 g de milieu synthétique sélectif dextrine (dextrine de maïs 220 g/kg, extrait de levure 5 g/kg, urée 2 g/kg, KH₂PO₄ 1 g/kg ainsi que des minéraux et des vitamines) est réalisée à 32°C. Les dextrines sont des hydrolysats d'amidon permettant de mimer le milieu réel.

Les souches de *S. cerevisiae* modifiées selon l'invention ont été préalablement propagées sur un milieu YPG (Yeast extract, Peptone, Glucose) pendant 24h à 30°C. Le pH initial du milieu de fermentation a été ajusté à 5.0 sans régulation. Le milieu de fermentation a ensuite été inoculé à un taux de 0.125 g équivalent matière sèche par kilogramme de milieu. Aucune enzyme d'hydrolyse exogène n'est ajoutée au milieu de fermentation. Des suivis de perte de masse ont été réalisés pendant 72 heures et sont illustrés figure 15, pour la série ER-GFD-8000 et figure 16 pour la série ER-GFD-48000.

Dans ce type de milieu de fermentation (dextrine), il y a peu de glucose libre à t0 (environ une dizaine de grammes). La souche ER, n'ayant pas d'enzyme pouvant hydrolyser les dextrines, elle consomme uniquement le glucose disponible et donc une faible perte de masse est mesurée (environ 5 g/kg) à partir de 14h jusqu'à la fin de la fermentation.

A partir des résultats de suivi de perte de masse présentés figure 15 et 16, les clones testés pour chaque série présentent des cinétiques de perte de masse quasi-identiques et semblent donc équivalent en performance fermentaire sur un milieu dextrine.

### Exemple 8 : Evaluation de la production d'activité enzymatique des souches

Les clones sélectionnés précédemment, ont été évaluées à 32°C sur le milieu industriel E140723-11 et comparées avec les souches témoin I-4998, I-4999 I-4899, I-4997, ER-SDG-4c et ER-SDG-8c. Il s'agit des clones suivants : ER-GAND-12020, ER-GAND 48084, ER-GFD-8044 et ER-GFD-48015.

La souche (déposée le 9 juillet 2015 à la CNCM sous le numéro I-4998) exprimant l'activité *STA1* issue de *S. cerevisiae* var. *diastaticus* permettait d'obtenir une cinétique d'hydrolyse des dextrines rapide mais incomplète, alors que la souche (I-4899) exprimant l'activité *GLAA* issue *d'A. niger* permettait d'obtenir une hydrolyse des dextrines satisfaisante mais avec une cinétique inférieure à I-4998.

Les souches ont été préalablement propagées sur un milieu riche pendant la nuit à 32°C. La fermentation a été réalisée à 32°C. Le pH initial du milieu de fermentation a été ajusté à 5.0 sans régulation. De l'urée a été ajoutée en fermentation (600 ppm). Le milieu de fermentation a ensuite été inoculé à un taux de 0.5 g équivalent matière sèche par kilogramme de milieu. On a mesuré au cours du temps de t=0 à t=66 h la perte de masse des réacteurs de fermentation.

Les résultats de pertes de masse obtenues au cours de la fermentation alcoolique sont présentés sur les Figure 17 et 18.

Pour les séries ER-GAND (I-5119 et I-5120), la figure 17 montre que les nouvelles souches permettent d'obtenir à la fois une hydrolyse des dextrines aussi rapide que les souches ER-SDG-4c et ER-SDG-8c et complète comme la souche I-4899, mais qu'elles sont aussi rapides que la souche I-4997. On peut en conclure que la production de glucoamylase de *Saccharomyces cerevisiae* var. *diastaticus* a toujours un effet stimulant sur l'activité hydrolytique de la glucoamylase *Aspergillus niger.*

Pour les séries ER-GFD (I-5121 et I-5122), la figure 18 montre que les nouvelles souches permettent d'obtenir à la fois une hydrolyse des dextrines aussi rapide que la souche I-ER-SDG4c et complète comme la souche I-4999, mais qu'elles sont également beaucoup plus rapides que la souche I-4999. On peut en conclure que la production de glucoamylase de *Saccharomyces cerevisiae* var. *diastaticus* a un effet stimulant sur l'activité hydrolytique de la glucoamylase *Saccharomycopsis fibuligera.* Ces souches ne font pas que de présenter une combinaison des caractéristiques de la glucoamylase de *Saccharomycopsis fibuligera* fongique et de celle de *Saccharomyces cerevisiae* var. *diastaticus* (c'est-à-dire avec un profil cinétique qui se situerait entre celui de la glucoamylase de *Saccharomycopsis fibuligera* et celui de la glucoamylase de *Saccharomyces cerevisiae* var. *diastaticus),* mais présentent un profil cinétique amélioré avec une accélération de la cinétique. Il y aurait donc un effet synergique entre la glucoamylase d'origine fongique et celle de *Saccharomyces cerevisiae* var. *diastaticus.*

### RESUME

La présente demande a pour objet des souches de levure de *Saccharomyces cerevisiae* améliorées, caractérisée en ce qu'elles co-expriment un gène codant une glucoamylase d'origine fongique choisie parmi une glucoamylase *d'Aspergillus niger* et une glucoamylase de *Saccharomycopsis fibuligera* et un gène codant la glucoamylase de *Saccharomyces cerevisiae* var. *diastaticus,* telle que définie dans la revendication 1. La présente invention concerne également une méthode d'obtention de ces souches de levure. Les souches de levure selon l'invention trouvent particulièrement leur intérêt dans la production de bioéthanol.

## Revendications

1. Souche de levure de *Saccharomyces cerevisiae,* **caractérisée en ce qu'**elle co-exprime :
- un gène codant une glucoamylase d'origine fongique choisie parmi une glucoamylase *d'Aspergillus niger* et une glucoamylase de *Saccharomycopsis fibuligera*; et
- un gène codant la glucoamylase de *Saccharomyces cerevisiae* var.
*diastaticus,*
où le gène codant la glucoamylase *d'Aspergillus niger* a la séquence nucléique SEQ ID NO : 1, le gène codant la glucoamylase de *Saccharomycopsis fibuligera* a la séquence nucléique SEQ ID NO : 17, et le gène codant la glucoamylase de *Saccharomyces cerevisiae var. diastaticus* a la séquence nucléique SEQ ID NO : 3, et **en ce qu'**elle contient :
- entre 2 et 10 copies, bornes incluses, du gène codant la glucoamylase d'origine fongique; et
- entre 2 et 10 copies, bornes incluses, du gène codant la glucoamylase de *Saccharomyces cerevisiae* var. *diastaticus.*

2. Souche de levure de *Saccharomyces cerevisiae* selon la revendication 1, **caractérisée en ce que** la glucoamylase de *Saccharomyces cerevisiae* var. *diastaticus* a la séquence protéique SEQ ID NO: 4.

3. Souche de levure de *Saccharomyces cerevisiae* selon la revendication 1 ou la revendication 2, **caractérisée en ce que** la glucoamylase d'origine fongique est une glucoamylase *d'Aspergillus niger* et a la séquence protéique SEQ ID NO: 2.

4. Souche de levure de *Saccharomyces cerevisiae* selon la revendication 1 ou la revendication 2, **caractérisée en ce que** la glucoamylse d'origine fongique est une glucoamylase de *Saccharomycopsis fibuligera* et a la séquence protéique SEQ ID NO 18.

5. Souche de levure de *Saccharomyces cerevisiae* selon l'une quelconque des revendications 1 à 4, **caractérisée en ce que** le gène codant une glucoamylase d'origine fongique et celui codant la glucoamylase de *Saccharomyces cerevisiae* var. *diastaticus* sont intégrés au sein du génome de ladite levure.

6. Souche de levure de *Saccharomyces cerevisiae* selon la revendication 1, **caractérisée en ce que** ladite souche est sélectionnée parmi la souche déposée le 6 août 2015 à la CNCM sous le numéro I-5005, la souche déposée le 9 Juillet 2015 à la CNCM sous le numéro I-4997, la souche déposée le 11 Août 2016 à la CNCM sous le numéro I-5119, la souche déposée le 11 Août 2016 à la CNCM sous le numéro I-5120, la souche déposée le 11 Août 2016 à la CNCM sous le numéro I-5121 et la souche déposée le 11 Août 2016 à la CNCM sous le numéro I-5122.

7. Souche de levure de *Saccharomyces cerevisiae,* **caractérisée en ce qu'**elle contient la séquence nucléique SEQ ID NO : 1 et la séquence nucléique SEQ ID NO : 3.

8. Souche de levure selon l'une quelconque des revendications 1 à 7, **caractérisée en ce que** la souche de levure contient 2, 3, 4, 5, 6, 7, 8, 9 ou 10 copies du gène codant la glucoamylase d'origine fongique et 2, 3, 4, 5, 6, 7, 8, 9 ou 10 copies du gène codant la glucoamylase de *Saccharomyces cerevisiae* var. *diastaticus.*

9. Souche de levure selon l'une quelconque des revendications 1 à 7, **caractérisée en ce que** la souche de levure contient 4 ou 8 copies du gène codant la glucoamylase *d'Aspergillus niger* et 3 ou 8 copies du gène codant la glucoamylase de *Saccharomyces cerevisiae var. diastaticus,* ou **en ce que** la souche de levure contient 4 copies du gène codant la glucoamylase de *Saccharomycopsis fibuligera et* 4 ou 8 copies du gène codant la glucoamylase de *Saccharomyces cerevisiae var. diastaticus.*

10. Méthode d'obtention d'une souche de levure comprenant les étapes suivantes :
a) modification génétique d'une levure de *Saccharomyces cerevisiae* de manière à la faire co-exprimer un gène codant une glucoamylase d'origine fongique choisie parmi une glucoamylase *d'Aspergillus niger* et une glucoamylase de *Saccharomycopsis fibuligera* et un gène codant la glucoamylase de *Saccharomyces cerevisiae* var. *diastaticus* et de manière à ce qu'elle contienne entre 2 et 10 copies, bornes incluses, du gène codant la glucoamylase d'origine fongique; et entre 2 et 10 copies, bornes incluses, du gène codant la glucoamylase de *Saccharomyces cerevisiae* var. *diastaticus;*
b) mise en culture et fermentation de la souche obtenue à l'étape a) sur un milieu dextrine;
c) sélection des souches présentant une cinétique de fermentation au moins égale ou supérieure à la souche déposée le 9 Juillet 2015 à la CNCM sous le numéro I-4999 dans les mêmes conditions,
dans laquelle le gène codant la glucoamylase *d'Aspergillus niger* a la séquence nuclétique SEQ ID NO : 1, le gène codant la glucoamylase de *Saccharomyces fibuligera* a la séquence nucléique SEQ ID NO : 17, et le gène codant la glucoamylase de *Saccharomyces cerevisiae var. diastaticus* a la séquence nucléique SEQ ID NO : 3.

11. Méthode d'obtention d'une souche de levure selon la revendication 10, **caractérisée en ce que** la souche de levure contient 2, 3, 4, 5, 6, 7, 8, 9 ou 10 copies du gène codant la glucoamylase d'origine fongique et 2, 3, 4, 5, 6, 7, 8, 9 ou 10 copies du gène codant la glucoamylase de *Saccharomyces cerevisiae* var. *diastaticus.*

12. Méthode d'obtention d'une souche de levure selon la revendication 10, **caractérisée en ce que** la souche de levure contient 4 ou 8 copies du gène codant la glucoamylase *d'Aspergillus niger* et 3 ou 8 copies du gène codant la glucoamylase de *Saccharomyces cerevisiae var. diastaticus* ou **en ce que** la souche de levure contient 4 copies du gène codant la glucoamylase de *Saccharomycopsis fibuligera et* 4 ou 8 copies du gène codant la glucoamylase de *Saccharomyces cerevisiae var. diastaticus.*

13. Procédé de production de bioéthanol à partir d'une biomasse comprenant les étapes suivantes :
a) préhydrolyse et liquéfaction de l'amidon de la biomasse;
b) mise en contact de l'amidon liquéfié obtenu à l'étape a) avec une levure telle que décrite dans l'une quelconque des revendications 1 à 9;
c) hydrolyse et fermentation de l'amidon liquéfié par ladite levure;
d) extraction de l'éthanol produit à l'étape c).

14. Procédé selon la revendication 13 comprenant en outre une étape b') d'ajout d'enzymes glucoamylases exogènes après l'étape b) et/ou au cours de l'étape c).

15. Utilisation d'une souche de levure selon l'une quelconque des revendications 1 à 9, pour la production de bioéthanol.

## Patentansprüche

1. Hefestamm von *Saccharomyces cerevisiae,* **dadurch gekennzeichnet, dass** er coexprimiert:
- ein Gen, das für eine Glucoamylase pilzlichen Ursprungs kodiert, ausgewählt aus einer Glucoamylase aus *Aspergillus niger* und einer Glucoamylase aus *Saccharomycopsis fibuligera*; und
- ein Gen, das für die Glucoamylase aus *Saccharomyces cerevisiae* var. *diastaticus* kodiert,
wobei das Gen, das für die Glucoamylase aus *Aspergillus niger* kodiert, die Nukleinsäuresequenz SEQ ID NO: 1 aufweist, das Gen, das für die Glucoamylase aus *Saccharomycopsis fibuligera* kodiert, die Nukleinsäuresequenz SEQ ID NO: 17 aufweist, und das Gen, das für die Glucoamylase aus *Saccharomyces cerevisiae var. diastaticus* kodiert, die Nukleinsäuresequenz SEQ ID NO: 3 aufweist,
und dass er enthält
- zwischen 2 und 10, Endwerte eingeschlossen, Kopien des Gens, das für die Glucoamylase pilzlichen Ursprungs kodiert; und
- zwischen 2 und 10, Endwerte eingeschlossen, Kopien des Gens, das für die Glucoamylase aus *Saccharomyces cerevisiae* var. *diastaticus* kodiert.

2. Hefestamm von *Saccharomyces cerevisiae* nach Anspruch 1, **dadurch gekennzeichnet, dass** die Glucoamylase aus *Saccharomyces cerevisiae* var. *diastaticus* die Proteinsequenz SEQ ID NO: 4 aufweist.

3. Hefestamm von *Saccharomyces cerevisiae* nach Anspruch 1 oder Anspruch 2,
**dadurch gekennzeichnet, dass** die Glucoamylase pilzlichen Ursprungs eine Glucoamylase aus *Aspergillus niger* ist und die Proteinsequenz SEQ ID NO: 2 aufweist.

4. Hefestamm von *Saccharomyces cerevisiae* nach Anspruch 1 oder Anspruch 2,
**dadurch gekennzeichnet, dass** die Glucoamylase pilzlichen Ursprungs eine Glucoamylase aus *Saccharomycopsis fibuligera* ist und die Proteinsequenz SEQ ID NO: 18 aufweist.

5. Hefestamm von *Saccharomyces cerevisiae* nach einem der Ansprüche 1 bis 4,
**dadurch gekennzeichnet, dass** das Gen, das für eine Glucoamylase pilzlichen Ursprungs kodiert, und das Gen, das für die Glucoamylase aus *Saccharomyces cerevisiae* var. *diastaticus* kodiert, in das Genom der Hefe integriert sind.

6. Hefestamm von *Saccharomyces cerevisiae* nach Anspruch 1, **dadurch gekennzeichnet, dass** der Stamm ausgewählt ist aus dem Stamm, der am 6. August 2015 bei der CNCM unter der Nummer I-5005 hinterlegt wurde, dem Stamm, der am 9. Juli 2015 bei der CNCM unter der Nummer I-4997 hinterlegt wurde, dem Stamm, der am 11. August 2016 bei der CNCM unter der Nummer I-5119 hinterlegt wurde, dem Stamm, der am 11. August 2016 bei der CNCM unter der Nummer I-5120 hinterlegt wurde, dem Stamm, der am 11. August 2016 bei der CNCM unter der Nummer I-5121 hinterlegt wurde und dem Stamm, der am 11. August 2016 bei der CNCM unter der Nummer I-5122 hinterlegt wurde.

7. Hefestamm von *Saccharomyces cerevisiae,* **dadurch gekennzeichnet, dass** er die Nukleinsäuresequenz SEQ ID NO: 1 und die Nukleinsäuresequenz SEQ ID NO: 3 enthält.

8. Hefestamm nach einem der Ansprüche 1 bis 7, **dadurch gekennzeichnet, dass** der Hefestamm 2, 3, 4, 5, 6, 7, 8, 9 oder 10 Kopien des Gens, das für die Glucoamylase pilzlichen Ursprungs kodiert, und 2, 3, 4, 5, 6, 7, 8, 9 oder 10 Kopien des Gens, das für die Glucoamylase aus *Saccharomyces cerevisiae* var. *diastaticus* kodiert, enthält.

9. Hefestamm nach einem der Ansprüche 1 bis 7, **dadurch gekennzeichnet, dass** der Hefestamm 4 oder 8 Kopien des Gens, das für die Glucoamylase aus *Aspergillus niger* kodiert, und 3 oder 8 Kopien des Gens, das für die Glucoamylase aus *Saccharomyces cerevisiae* var. *diastaticus* kodiert, enthält, oder dass der Hefestamm 4 Kopien des Gens, das für die Glucoamylase aus *Saccharomycopsis fibuligera* kodiert und 4 oder 8 Kopien des Gens, das für die Glucoamylase aus *Saccharomyces cerevisiae* var. *diastaticus* kodiert, enthält.

10. Verfahren zur Gewinnung eines Hefestammes, umfassend die folgenden Schritte:
a) genetische Veränderung einer *Saccharomyces cerevisiae-*Hefe, um sie zur Coexpression eines Gens, das für eine Glucoamylase pilzlichen Ursprungs kodiert, ausgewählt aus einer Glucoamylase aus *Aspergillus niger* und einer Glucoamylase aus *Saccharomycopsis fibuligera;* und eines Gens, das für die Glucoamylase aus *Saccharomyces cerevisiae* var. *diastaticus* kodiert, zu veranlassen, und zwar so, dass sie zwischen 2 und 10 Kopien, Endwerte eingeschlossen, des Gens, das für die Glucoamylase pilzlichen Ursprungs kodiert, und zwischen 2 und 10 Kopien, Endwerte eingeschlossen, des Gens, das für die Glucoamylase aus *Saccharomyces cerevisiae* var. *diastaticus* kodiert, enthält;
b) Kultivierung und Fermentation des in Schritt a) erhaltenen Stammes auf einem Dextrinmedium;
c) Auswahl von Stämmen mit einer Fermentationskinetik, die mindestens genauso gut wie oder besser als die des am 9. Juli 2015 bei der CNCM unter der Nummer I-4999 hinterlegten Stammes unter denselben Bedingungen ist,
wobei das Gen, das für die Glucoamylase aus *Aspergillus niger* kodiert, die Nukleinsäuresequenz SEQ ID NO: 1 aufweist, das Gen, das für die Glucoamylase aus *Saccharomycopsis fibuligera* kodiert, die Nukleinsäuresequenz SEQ ID NO: 17 aufweist, und das Gen, das für die Glucoamylase aus *Saccharomyces cerevisiae* var. *diastaticus* kodiert, die Nukleinsäuresequenz SEQ ID NO: 3 aufweist.

11. Verfahren zur Gewinnung eines Hefestamms nach Anspruch 10, **dadurch gekennzeichnet, dass** der Hefestamm 2, 3, 4, 5, 6, 7, 8, 9 oder 10 Kopien des für die Glucoamylase pilzlichen Ursprungs kodierenden Gens und 2, 3, 4, 5, 6, 7, 8, 9 oder 10 Kopien des für die Glucoamylase aus *Saccharomyces cerevisiae* var. *diastaticus* kodierenden Gens enthält.

12. Verfahren zur Gewinnung eines Hefestamms nach Anspruch 10, **dadurch gekennzeichnet, dass** der Hefestamm 4 oder 8 Kopien des Gens, das für die Glucoamylase aus *Aspergillus niger* kodiert und 3 oder 8 Kopien des Gens, das für die Glucoamylase aus *Saccharomyces cerevisiae* var. *diastaticus* kodiert, enthält, oder dadurch, dass der Hefestamm 4 Kopien des Gens, das für die Glucoamylase aus *Saccharomycopsis fibuligera* kodiert und 4 oder 8 Kopien des Gens, das für die Glucoamylase aus *Saccharomyces cerevisiae* var. *diastaticus* kodiert, enthält.

13. Verfahren zur Herstellung von Bioethanol aus Biomasse, umfassend die folgenden Schritte:
a) Vorhydrolyse und Verflüssigung der Stärke der Biomasse;
b) Inkontaktbringen der in Schritt a) erhaltenen verflüssigten Stärke mit einer Hefe, wie in einem der Ansprüche 1 bis 9 beschrieben;
c) Hydrolyse und Fermentation der verflüssigten Stärke durch die Hefe;
d) Extraktion des in Schritt c) hergestellten Ethanols.

14. Verfahren nach Anspruch 13, ferner umfassend einen Schritt b') der Zugabe exogener Glucoamylase-Enzyme nach Schritt b) und/oder während Schritt c).

15. Verwendung eines Hefestamms nach einem der Ansprüche 1 bis 9 zur Herstellung von Bioethanol.

## Claims

1. *Saccharomyces cerevisiae* yeast strain, **characterised in that** it coexpressses:
- a gene encoding a glucoamylase of fungal origin selected from a glucoamylase of *Aspergillus niger* glucoamylase and a glucoamylase of *Saccharomycopsis fibuligera,* and
- a gene encoding the glucoamylase of *Saccharomyces cerevisiae* var.
*diastaticus,*
wherein the gene encoding the glucoamylase of *Aspergillus niger* has the nucleic sequence SEQ ID NO: 1, the gene encoding the glucoamylase of *Saccharomycopsis fibuligera* has the nucleic sequence SEQ ID NO: 17, and the gene encoding the glucoamylase of *Saccharomyces cerevisiae* var. *diastaticus* has the nucleic sequence SEQ ID NO: 3,
and **in that** it contains:
- between 2 and 10 copies, inclusive, of the gene encoding the glucoamylase of fungal origin; and
- between 2 and 10 copies, inclusive, of the gene encoding the glucoamylase of *Saccharomyces cerevisiae* var. *diastaticus.*

2. *Saccharomyces cerevisiae* yeast strain according to claim 1, **characterised in that** the glucoamylase of *Saccharomyces cerevisiae* var. *diastaticus* has the protein sequence SEQ ID NO: 4.

3. *Saccharomyces cerevisiae* yeast strain according to claim 1 or claim 2, **characterised in that** the glucoamylase of fungal origin is a glucoamylase of *Aspergillus niger* and has the protein sequence SEQ ID NO: 2.

4. *Saccharomyces cerevisiae* yeast strain according to claim 1 or claim 2, **characterised in that** the glucoamylase of fungal origin is a glucoamylase of *Saccharomycopsis fibuligera* and has the protein sequence SEQ ID NO: 18.

5. *Saccharomyces cerevisiae* yeast strain according to any one of claims 1 to 4, **characterised in that** the gene encoding a glucoamylase of fungal origin and the gene encoding the glucoamylase of *Saccharomyces cerevisiae* var. *diastaticus* are integrated in the genome of said yeast.

6. *Saccharomyces cerevisiae* yeast strain according to claim 1, **characterised in that** said strain is selected from the strain deposited on 6 August 2016 at the CNCM under the number I-5005, the strain deposited on 9 July 2015 at the CNCM under the number I-4997, the strain deposited on 11 August 2016 at the CNCM under the number I-5119, the strain deposited on 11 August 2016 at the CNCM under the number I-5120, the strain deposited on 11 August 2016 at the CNCM under the number I-5121 and the strain deposited on 11 August 2016 at the CNCM under the number I-5122.

7. *Saccharomyces cerevisiae* yeast strain, **characterised in that** it contains the nucleic sequence SEQ ID NO: 1 and the nucleic sequence SEQ ID NO: 3.

8. Yeast strain according to any one of claims 1 to 7, **characterised in that** the yeast strain contains 2, 3, 4, 5, 6, 7, 8, 9 or 10 copies of the gene encoding the glucoamylase of fungal origin and 2, 3, 4, 5, 6, 7, 8, 9 or 10 copies of the gene encoding the glucoamylase of *Saccharomyces cerevisiae* var. *diastaticus.*

9. Yeast strain according to any one of claims 1 to 7, **characterised in that** the yeast strain contains 4 or 8 copies of the gene encoding the glucoamylase of *Aspergillus niger* and 3 or 8 copies of the gene encoding the glucoamylase of *Saccharomyces cerevisiae* var. *diastaticus,* or **in that** the yeast strain contains 4 copies of the gene encoding the glucoamylase of *Saccharomycopsis fibuligera* and 4 or 8 copies of the gene encoding the glucoamylase of *Saccharomyces cerevisiae* var. *diastaticus.*

10. Method for obtaining a yeast strain, comprising the following steps:
a) genetic modification of a *Saccharomyces cerevisiae* yeast so as to coexpress a gene encoding a glucoamylase of fungal origin selected from a glucoamylase of *Aspergillus niger* and a glucoamylase of *Saccharomycopsis fibuligera* and a gene encoding the glucoamylase of *Saccharomyces cerevisiae* var. *diastaticus* and so that it contains between 2 and 10 copies, inclusive, of the gene encoding the glucoamylase of fungal origin, and between 2 and 10 copies, inclusive, of the gene encoding the glucoamylase of *Saccharomyces cerevisiae* var. *diastaticus;*
b) culturing and fermentation of the strain obtained at step a) on a dextrin medium;
c) selection of the strains having a fermentation kinetics at least equal to or greater than the strain deposited on 9 July 2015 at the CNCM under the number I-4999 under the same conditions,
wherein the gene encoding the glucoamylase of *Aspergillus niger* has the nucleic sequence SEQ ID NO: 1, the gene encoding the glucoamylase of *Saccharomyces fibuligera* has the nucleic sequence SEQ ID NO: 17, and the gene encoding the glucoamylase of *Saccharomyces cerevisiae* var. *diastaticus* has the nucleic sequence SEQ ID NO: 3.

11. Method for obtaining a yeast strain according to claim 10, **characterised in that** the yeast strain contains 2, 3, 4, 5, 6, 7, 8, 9 or 10 copies of the gene encoding the glucoamylase of fungal origin and 2, 3, 4, 5, 6, 7, 8, 9 or 10 copies of the gene encoding the glucoamylase of *Saccharomyces cerevisiae* var. *diastaticus.*

12. Method for obtaining a yeast strain according to claim 10, **characterised in that** the yeast strain contains 4 or 8 copies of the gene encoding the glucoamylase of *Aspergillus niger* and 3 or 8 copies of the gene encoding the glucoamylase of *Saccharomyces cerevisiae* var. *diastaticus,* or **in that** the yeast strain contains 4 copies of the gene encoding the glucoamylase of *Saccharomycopsis fibuligera* and 4 or 8 copies of the gene encoding the glucoamylase of *Saccharomyces cerevisiae* var. *diastaticus.*

13. Method for producing bioethanol from a biomass, comprising the following steps:
a) prehydrolysis and liquefaction of the starch of the biomass;
b) putting the liquefied starch obtained at step a) in contact with a yeast as described in any one of claims 1 to 9;
c) hydrolysis and fermentation of the liquefied starch by said yeast;
d) extracting the ethanol produced at step c).

14. Method according to claim 13, furthermore comprising a step b') of adding exogenous glucoamylase enzymes after step b) and/or during step c).

15. Use of a yeast strain according to any one of claims 1 to 9, for producing bioethanol.


### SEQUENCE LISTING

<110> LESAFFRE
<120> SOUCHES DE LEVURE CO-EXPRIMANT DES GLUCOAMYLASES EXOGENES, LEUR PROCEDE D'OBTENTION ET LEUR UTILISATION POUR PRODUIRE DU BIOETHANOL
<130> BFF150287
<160> 22
<170> PatentIn version 3.5
<210> 1
   <211> 1923
   <212> DNA
   <213> Aspergillus niger
<400> 1
   atgtccttta gatcattgct tgcactgtct ggtttagttt gcactggact tgccaatgtc      60
   atctctaaga gagcgacact agattcctgg ttaagtaacg aagccactgt tgcacgtaca     120
   gccatactta acaacatagg tgccgatgga gcatgggtta gtggcgcaga ttcggggata     180
   gtcgttgcca gtccatcgac tgataatcct gactactttt atacctggac ccgtgatagt     240
   gggttggtgc tgaaaacatt ggtagacctt ttcagaaacg gtgatacgtc cttgctatca     300
   accatagaga attacattag cgcacaagct attgtacaag gcatttccaa tccgagtggc     360
   gatctaagtt caggcgctgg cttgggtgaa cccaagttca atgtcgatga aactgcatat     420
   acgggatcat ggggtagacc acaaagagat ggtccagctc taagagcaac tgccatgatt     480
   ggatttggcc aatggctttt ggataatggc tacactagta cagccacaga cattgtttgg     540
   cctttagtca gaaatgacct atcttatgtg gctcaatatt ggaaccaaac aggttatgac     600
   ttatgggaag aagtcaatgg ttcttctttc tttacaattg ccgtacagca tcgtgcactg     660
   gtggaaggat cggctttcgc cactgccgta ggttcctcat gtagttggtg tgattcacaa     720
   gcgccagaga ttctatgcta tttgcagagc ttctggacag ggagttttat cttagccaac     780
   ttcgatagct ctagatccgg gaaagatgct aataccctat taggctcaat acacacgttt     840
   gaccctgaag ctgcttgtga cgattctaca tttcaaccgt gttctcccag agctttggca     900
   aaccataaag aagttgttga ctcttttagg tctatctaca ccttaaacga cggtttgtcg     960
   gattcagaag ctgtggcagt tgggaggtat ccggaggata cgtactacaa tggtaatcct    1020
   tggttccttt gcactttggc agccgcggag cagttatatg atgcgttata tcaatgggat    1080
   aagcagggtt ccttagaggt aactgatgtg tcgctggact tctttaaagc gctgtattca    1140
   gatgctgcta ccggtacgta ttcttcgtca tcttcaacct attccagcat tgtggatgct    1200
   gtcaagactt ttgcagacgg atttgtcagt atagttgaga ctcatgcagc ttctaatggt    1260
   tctatgtccg aacagtacga caaaagcgat ggtgaacaat tgtcagcaag agacttgacc    1320
   tggtcttatg cagccttgtt aacagccaac aataggagaa atagcgttgt tccagctagt     1380
   tggggagaaa catccgcgtc atcagttcca ggaacgtgtg ctgctacttc agctattggt     1440
   acatattctt cagttacagt cacctcttgg ccttcgatag tagctactgg aggaactact     1500
   acgaccgcta ctcctacagg tagcggttct gtgacttcca cctcaaagac aactgctact     1560
   gctagcaaaa catctacctc tacttcgtcc acatcatgca ctaccccaac tgcagtcgca     1620
   gttacgtttg atttgacagc tactacaacg tacggggaaa acatttactt ggtaggtagc     1680
   atcagtcaat tgggcgactg ggaaaccagc gatggtattg cattgagtgc agataaatac     1740
   acttcctctg atccattatg gtatgttacc gttacgttac cagctggtga gtcctttgaa     1800
   tacaagttca tcagaatcga gagtgatgac tctgtggaat gggaatctga tcccaataga     1860
   gaatacacag tacctcaagc gtgtggtaca tcaacagcca ccgtaactga tacttggagg     1920
   taa                                                                   1923
<210> 2
   <211> 640
   <212> PRT
   <213> Aspergillus niger
<400> 2
   Met Ser Phe Arg Ser Leu Leu Ala Leu Ser Gly Leu Val Cys Thr Gly
   1               5                   10                  15
   Leu Ala Asn Val Ile Ser Lys Arg Ala Thr Leu Asp Ser Trp Leu Ser
               20                  25                  30
   Asn Glu Ala Thr Val Ala Arg Thr Ala Ile Leu Asn Asn Ile Gly Ala
           35                  40                  45
   Asp Gly Ala Trp Val Ser Gly Ala Asp Ser Gly Ile Val Val Ala Ser
       50                  55                  60
   Pro Ser Thr Asp Asn Pro Asp Tyr Phe Tyr Thr Trp Thr Arg Asp Ser
   65                  70                  75                  80
   Gly Leu Val Leu Lys Thr Leu Val Asp Leu Phe Arg Asn Gly Asp Thr
                   85                  90                  95
   Ser Leu Leu Ser Thr Ile Glu Asn Tyr Ile Ser Ala Gln Ala Ile Val
               100                 105                 110
   Gln Gly Ile Ser Asn Pro Ser Gly Asp Leu Ser Ser Gly Ala Gly Leu
           115                 120                 125
   Gly Glu Pro Lys Phe Asn Val Asp Glu Thr Ala Tyr Thr Gly Ser Trp
       130                 135                 140
   Gly Arg Pro Gln Arg Asp Gly Pro Ala Leu Arg Ala Thr Ala Met Ile
   145                 150                 155                 160
   Gly Phe Gly Gln Trp Leu Leu Asp Asn Gly Tyr Thr Ser Thr Ala Thr
                   165                 170                 175
   Asp Ile Val Trp Pro Leu Val Arg Asn Asp Leu Ser Tyr Val Ala Gln
               180                 185                 190
   Tyr Trp Asn Gln Thr Gly Tyr Asp Leu Trp Glu Glu Val Asn Gly Ser
           195                 200                 205
   Ser Phe Phe Thr Ile Ala Val Gln His Arg Ala Leu Val Glu Gly Ser
       210                 215                 220
   Ala Phe Ala Thr Ala Val Gly Ser Ser Cys Ser Trp Cys Asp Ser Gln
   225                 230                 235                 240
   Ala Pro Glu Ile Leu Cys Tyr Leu Gln Ser Phe Trp Thr Gly Ser Phe
                   245                 250                 255
   Ile Leu Ala Asn Phe Asp Ser Ser Arg Ser Gly Lys Asp Ala Asn Thr
               260                 265                 270
   Leu Leu Gly Ser Ile His Thr Phe Asp Pro Glu Ala Ala Cys Asp Asp
           275                 280                 285
   Ser Thr Phe Gln Pro Cys Ser Pro Arg Ala Leu Ala Asn His Lys Glu
       290                 295                 300
   Val Val Asp Ser Phe Arg Ser Ile Tyr Thr Leu Asn Asp Gly Leu Ser
   305                 310                 315                 320
   Asp Ser Glu Ala Val Ala Val Gly Arg Tyr Pro Glu Asp Thr Tyr Tyr
                   325                 330                 335
   Asn Gly Asn Pro Trp Phe Leu Cys Thr Leu Ala Ala Ala Glu Gln Leu
               340                 345                 350
   Tyr Asp Ala Leu Tyr Gln Trp Asp Lys Gln Gly Ser Leu Glu Val Thr
           355                 360                 365
   Asp Val Ser Leu Asp Phe Phe Lys Ala Leu Tyr Ser Asp Ala Ala Thr
       370                 375                 380
   Gly Thr Tyr Ser Ser Ser Ser Ser Thr Tyr Ser Ser Ile Val Asp Ala
   385                 390                 395                 400
   Val Lys Thr Phe Ala Asp Gly Phe Val Ser Ile Val Glu Thr His Ala
                   405                 410                 415
   Ala Ser Asn Gly Ser Met Ser Glu Gln Tyr Asp Lys Ser Asp Gly Glu
               420                 425                 430
   Gln Leu Ser Ala Arg Asp Leu Thr Trp Ser Tyr Ala Ala Leu Leu Thr
           435                 440                 445
   Ala Asn Asn Arg Arg Asn Ser Val Val Pro Ala Ser Trp Gly Glu Thr
       450                 455                 460
   Ser Ala Ser Ser Val Pro Gly Thr Cys Ala Ala Thr Ser Ala Ile Gly
   465                 470                 475                 480
   Thr Tyr Ser Ser Val Thr Val Thr Ser Trp Pro Ser Ile Val Ala Thr
                   485                 490                 495
   Gly Gly Thr Thr Thr Thr Ala Thr Pro Thr Gly Ser Gly Ser Val Thr
               500                 505                 510
   Ser Thr Ser Lys Thr Thr Ala Thr Ala Ser Lys Thr Ser Thr Ser Thr
           515                 520                 525
   Ser Ser Thr Ser Cys Thr Thr Pro Thr Ala Val Ala Val Thr Phe Asp
       530                 535                 540
   Leu Thr Ala Thr Thr Thr Tyr Gly Glu Asn Ile Tyr Leu Val Gly Ser
   545                 550                 555                 560
   Ile Ser Gln Leu Gly Asp Trp Glu Thr Ser Asp Gly Ile Ala Leu Ser
                   565                 570                 575
   Ala Asp Lys Tyr Thr Ser Ser Asp Pro Leu Trp Tyr Val Thr Val Thr
               580                 585                 590
   Leu Pro Ala Gly Glu Ser Phe Glu Tyr Lys Phe Ile Arg Ile Glu Ser
           595                 600                 605
   Asp Asp Ser Val Glu Trp Glu Ser Asp Pro Asn Arg Glu Tyr Thr Val
       610                 615                 620
   Pro Gln Ala Cys Gly Thr Ser Thr Ala Thr Val Thr Asp Thr Trp Arg
   625                 630                 635                 640
<210> 3
   <211> 2418
   <212> DNA
   <213> Saccharomyces cerevisiae var. diastaticus
<400> 3
   atgcaaagac catttctact cgcttatttg gtcctttcgc ttctatttaa ctcagctttg      60
   ggttttccaa ctgcactagt tcctagagga tcctcctcta gcaacatcac ttcctccggt     120
   ccatcttcaa ctccattcag ctctgctact gaaagctttt ctactggcac tactgtcact     180
   ccatcatcat ccaaataccc tggcagtaaa acagaaactt ctgtttcttc tacaaccgaa     240
   actaccattg ttccaactac aactacgact tctgtcataa caccatcaac aaccactatt     300
   accactacgg tttgctctac aggaacaaac tctgccggtg aaactacttc tggatgctct     360
   ccaaagacca ttacaactac tgttccatgt tcaaccagtc caagcgaaac cgcatcggaa     420
   tcaacaacca cttcacctac cacacctgta actacagttg tctcaaccac cgtcgttact     480
   actgagtatt ctactagtac aaaacaaggt ggtgaaatta caactacatt tgtcaccaaa     540
   aacattccaa ccacttacct aactacaatt gctccaactt catcagtcac tacggttacc     600
   aatttcaccc caaccactat tactactacg gtttgctcta caggaacaaa ctctgccggt     660
   gaaactacct ctggatgctc tccaaagact gtcacaacaa ctgttccttg ttcaactggt     720
   actggcgaat acactactga agctaccgcc cctgttacaa cagctgtcac aaccaccgtt     780
   gttaccactg aatcctctac gggtactaac tccgctggta agacgacaac tagttacaca     840
   acaaagtctg taccaaccac ctatgtattt gactttggca agggcattct cgatcaaagc     900
   tgcggcggtg tattttcaaa caacggctct tcgcaagtgc agctgcggga tgtagtcttg     960
   atgaatggga cagtggtata cgattcaaac ggcgcttggg acagtagtcc gctggaggag    1020
   tggctccagc gacagaaaaa agtttccatc gaaagaatat ttgaaaatat tgggcccagc    1080
   gccgtgtatc cgtctatttt gcctggggtc gtgattgcgt caccatcgca aacgcatcca    1140
   gactacttct accaatggat aagggacagc gcgttgacga taaacagtat tgtctctcat    1200
   tctgcggacc cggcaataga gacgttattg cagtacctga acgtttcatt ccacttgcaa    1260
   agaaccaaca acacattggg cgctggcatt ggttacacta acgatacagt ggctttggga    1320
   gaccctaagt ggaacgtcga caacacggct ttcacggaac cttggggtcg tcctcaaaac    1380
   gatggccctg ctcttcgaag cattgccatc ttaaaaatca tcgactacat caagcaatct    1440
   ggcactgatc tgggggccaa gtacccattc cagtccaccg cagatatctt tgatgatatt    1500
   gtacgttggg acctgaggtt cattattgac cactggaatt cttccggatt tgatctatgg    1560
   gaggaagtca atggcatgca tttctttact ttactggtac aactgtctgc agtggacagg    1620
   tcgctgtcgt attttaacgc ctcagaacgg tcgtctccct ttgttgaaga attgcgtcag    1680
   acacgccggg acatctccaa gtttttagtg gaccctgcga atgggtttat caacggcaag     1740
   tacaattata ttgttgagac acccatgatt gccgacacat tgagatccgg actggacata     1800
   tccactttat tagctgcgaa caccgtccac gatgcgccat ctgcttccca tcttccgttc     1860
   gatatcaatg accctgccgt cctgaacacg ttgcaccatt tgatgttgca catgcgttcg     1920
   atatacccca tcaacgatag ctccaaaaat gcaacgggta ttgccctggg ccggtatcct     1980
   gaggacgtat atgatggata tggcgttggc gagggaaatc cctgggtcct ggccacgtgt     2040
   gccgcttcaa caacgcttta tcagctcatt tacagacaca tctctgagca gcatgacttg     2100
   gttgtcccaa tgaacaacga ttgttcgaac gcattttgga gcgagctggt attctccaac     2160
   ctcacgactt tgggaaatga cgaaggctat ttgattttgg agttcaatac acctgccttc     2220
   aatcaaacca tacaaaaaat cttccaacta gctgattcat ttcttggtca agctgaaagc     2280
   cacgtgggaa cagacgggga actaagtgaa caatttaaca aatacacagg gtttatgcag     2340
   ggtgcccaac accttacctg gtcctatact tcattctggg atgcctatca aataagacaa     2400
   gaagttttac agagtttg                                                   2418
<210> 4
   <211> 806
   <212> PRT
   <213> Saccharomyces cerevisiae var. diastaticus
<400> 4
   Met Gln Arg Pro Phe Leu Leu Ala Tyr Leu Val Leu Ser Leu Leu Phe
   1               5                   10                  15
   Asn Ser Ala Leu Gly Phe Pro Thr Ala Leu Val Pro Arg Gly Ser Ser
               20                  25                  30
   Ser Ser Asn Ile Thr Ser Ser Gly Pro Ser Ser Thr Pro Phe Ser Ser
           35                  40                  45
   Ala Thr Glu Ser Phe Ser Thr Gly Thr Thr Val Thr Pro Ser Ser Ser
       50                  55                  60
   Lys Tyr Pro Gly Ser Lys Thr Glu Thr Ser Val Ser Ser Thr Thr Glu
   65                  70                  75                  80
   Thr Thr Ile Val Pro Thr Thr Thr Thr Thr Ser Val Ile Thr Pro Ser
                   85                  90                  95
   Thr Thr Thr Ile Thr Thr Thr Val Cys Ser Thr Gly Thr Asn Ser Ala
               100                 105                 110
   Gly Glu Thr Thr Ser Gly Cys Ser Pro Lys Thr Ile Thr Thr Thr Val
           115                 120                 125
   Pro Cys Ser Thr Ser Pro Ser Glu Thr Ala Ser Glu Ser Thr Thr Thr
       130                 135                 140
   Ser Pro Thr Thr Pro Val Thr Thr Val Val Ser Thr Thr Val Val Thr
   145                 150                 155                 160
   Thr Glu Tyr Ser Thr Ser Thr Lys Gln Gly Gly Glu Ile Thr Thr Thr
                   165                 170                 175
   Phe Val Thr Lys Asn Ile Pro Thr Thr Tyr Leu Thr Thr Ile Ala Pro
               180                 185                 190
   Thr Ser Ser Val Thr Thr Val Thr Asn Phe Thr Pro Thr Thr Ile Thr
           195                 200                 205
   Thr Thr Val Cys Ser Thr Gly Thr Asn Ser Ala Gly Glu Thr Thr Ser
       210                 215                 220
   Gly Cys Ser Pro Lys Thr Val Thr Thr Thr Val Pro Cys Ser Thr Gly
   225                 230                 235                 240
   Thr Gly Glu Tyr Thr Thr Glu Ala Thr Ala Pro Val Thr Thr Ala Val
                   245                 250                 255
   Thr Thr Thr Val Val Thr Thr Glu Ser Ser Thr Gly Thr Asn Ser Ala
               260                 265                 270
   Gly Lys Thr Thr Thr Ser Tyr Thr Thr Lys Ser Val Pro Thr Thr Tyr
           275                 280                 285
   Val Phe Asp Phe Gly Lys Gly Ile Leu Asp Gln Ser Cys Gly Gly Val
       290                 295                 300
   Phe Ser Asn Asn Gly Ser Ser Gln Val Gln Leu Arg Asp Val Val Leu
   305                 310                 315                 320
   Met Asn Gly Thr Val Val Tyr Asp Ser Asn Gly Ala Trp Asp Ser Ser
                   325                 330                 335
   Pro Leu Glu Glu Trp Leu Gln Arg Gln Lys Lys Val Ser Ile Glu Arg
               340                 345                 350
   Ile Phe Glu Asn Ile Gly Pro Ser Ala Val Tyr Pro Ser Ile Leu Pro
           355                 360                 365
   Gly Val Val Ile Ala Ser Pro Ser Gln Thr His Pro Asp Tyr Phe Tyr
       370                 375                 380
   Gln Trp Ile Arg Asp Ser Ala Leu Thr Ile Asn Ser Ile Val Ser His
   385                 390                 395                 400
   Ser Ala Asp Pro Ala Ile Glu Thr Leu Leu Gln Tyr Leu Asn Val Ser
                   405                 410                 415
   Phe His Leu Gln Arg Thr Asn Asn Thr Leu Gly Ala Gly Ile Gly Tyr
               420                 425                 430
   Thr Asn Asp Thr Val Ala Leu Gly Asp Pro Lys Trp Asn Val Asp Asn
           435                 440                 445
   Thr Ala Phe Thr Glu Pro Trp Gly Arg Pro Gln Asn Asp Gly Pro Ala
       450                 455                 460
   Leu Arg Ser Ile Ala Ile Leu Lys Ile Ile Asp Tyr Ile Lys Gln Ser
   465                 470                 475                 480
   Gly Thr Asp Leu Gly Ala Lys Tyr Pro Phe Gln Ser Thr Ala Asp Ile
                   485                 490                 495
   Phe Asp Asp Ile Val Arg Trp Asp Leu Arg Phe Ile Ile Asp His Trp
               500                 505                 510
   Asn Ser Ser Gly Phe Asp Leu Trp Glu Glu Val Asn Gly Met His Phe
           515                 520                 525
   Phe Thr Leu Leu Val Gln Leu Ser Ala Val Asp Arg Ser Leu Ser Tyr
       530                 535                 540
   Phe Asn Ala Ser Glu Arg Ser Ser Pro Phe Val Glu Glu Leu Arg Gln
   545                 550                 555                 560
   Thr Arg Arg Asp Ile Ser Lys Phe Leu Val Asp Pro Ala Asn Gly Phe
                   565                 570                 575
   Ile Asn Gly Lys Tyr Asn Tyr Ile Val Glu Thr Pro Met Ile Ala Asp
               580                 585                 590
   Thr Leu Arg Ser Gly Leu Asp Ile Ser Thr Leu Leu Ala Ala Asn Thr
           595                 600                 605
   Val His Asp Ala Pro Ser Ala Ser His Leu Pro Phe Asp Ile Asn Asp
       610                 615                 620
   Pro Ala Val Leu Asn Thr Leu His His Leu Met Leu His Met Arg Ser
   625 630 635 640
   Ile Tyr Pro Ile Asn Asp Ser Ser Lys Asn Ala Thr Gly Ile Ala Leu
   645 650 655
   Gly Arg Tyr Pro Glu Asp Val Tyr Asp Gly Tyr Gly Val Gly Glu Gly
   660 665 670
   Asn Pro Trp Val Leu Ala Thr Cys Ala Ala Ser Thr Thr Leu Tyr Gln
   675 680 685
   Leu Ile Tyr Arg His Ile Ser Glu Gln His Asp Leu Val Val Pro Met
   690 695 700
   Asn Asn Asp Cys Ser Asn Ala Phe Trp Ser Glu Leu Val Phe Ser Asn
   705 710 715 720
   Leu Thr Thr Leu Gly Asn Asp Glu Gly Tyr Leu Ile Leu Glu Phe Asn
   725 730 735
   Thr Pro Ala Phe Asn Gln Thr Ile Gln Lys Ile Phe Gln Leu Ala Asp
   740 745 750
   Ser Phe Leu Gly Gln Ala Glu Ser His Val Gly Thr Asp Gly Glu Leu
   755 760 765
   Ser Glu Gln Phe Asn Lys Tyr Thr Gly Phe Met Gln Gly Ala Gln His
   770 775 780
   Leu Thr Trp Ser Tyr Thr Ser Phe Trp Asp Ala Tyr Gln Ile Arg Gln
   785 790 795 800
   Glu Val Leu Gln Ser Leu
   805
<210> 5
   <211> 71
   <212> DNA
   <213> Artificial Sequence
<220>
   <223> Amorce
<400> 5
   tctgatggct aacggtgaaa ttaaagacat cgcaaacgtc acggctaact tgaagcttcg 60
tacgctgcag g 71
<210> 6
   <211> 52
   <212> DNA
   <213> Artificial Sequence
<220>
   <223> Amorce
<400> 6
   tcactgtacg gtgagaacgt agatggtgtg cgcataggcc actagtggat ct 52
<210> 7
   <211> 50
   <212> DNA
   <213> Artificial Sequence
<220>
   <223> Amorce
<400> 7
   cacaccatct acgttctcac cgtacagtga gcataaccgc tagagtactt 50
<210> 8
   <211> 50
   <212> DNA
   <213> Artificial Sequence
<220>
   <223> Amorce
<400> 8
   ttacgtagac tgagtagcaa cggttgagga cagcttgcaa attaaagcct 50
<210> 9
   <211> 50
   <212> DNA
   <213> Artificial Sequence
<220>
   <223> Amorce
<400> 9
   tcctcaaccg ttgctactca gtctacgtaa gcataaccgc tagagtactt 50
<210> 10
   <211> 50
   <212> DNA
   <213> Artificial Sequence
<220>
   <223> Amorce
<400> 10
   tcagtagcac agagaagtgt aggagtgtag cagcttgcaa attaaagcct 50
<210> 11
   <211> 50
   <212> DNA
   <213> Artificial Sequence
<220>
   <223> Amorce
<400> 11
   ctacactcct acacttctct gtgctactga gcataaccgc tagagtactt 50
<210> 12
   <211> 50
   <212> DNA
   <213> Artificial Sequence
<220>
   <223> Amorce
<400> 12
   ttaggataca tgcagtagac gaggtaagca cagcttgcaa attaaagcct 50
<210> 13
   <211> 50
   <212> DNA
   <213> Artificial Sequence
<220>
   <223> Amorce
<400> 13
   tgcttacctc gtctactgca tgtatcctaa gcataaccgc tagagtactt 50
<210> 14
   <211> 70
   <212> DNA
   <213> Artificial Sequence
<220>
   <223> Amorce
<400> 14
   acatacttgc aatttataca gtgatgaccg ctgaatttgt atcttccata cagcttgcaa 60
attaaagcct 70
<210> 15
   <211> 70
   <212> DNA
   <213> Artificial Sequence
<220>
   <223> Amorce
<400> 15
   taagggatat agaagcaaat agttgtcagt gcaatccttc aagacgattg gcataaccgc 60
tagagtactt 70
<210> 16
   <211> 70
   <212> DNA
   <213> Artificial Sequence
<220>
   <223> Amorce
<400> 16
   cacatataca gcatcggaat gagggaaatt tgttcatatc gtcgttgagt cagcttgcaa 60
attaaagcct 70
<210> 17
   <211> 1545
   <212> DNA
   <213> Saccharomycopsis fibuligera
<400> 17
   atgatcagat tgaccgtttt cttgaccgct gtttttgctg ctgttgcttc ttgtgttcca      60
   gttgaattgg ataagagaaa caccggtcat ttccaagctt attctggtta taccgttgct     120
   agatctaact tcacccaatg gattcatgaa caaccagctg tttcttggta ctacttgttg     180
   caaaacatcg attacccaga aggtcaattc aaatctgcta aaccaggtgt tgttgttgct     240
   tctccatcta catctgaacc agattacttc taccaatgga ctagagatac cgctattacc     300
   ttcttgtcct tgattgctga agttgaagat cattctttct ccaacactac cttggctaag     360
   gttgtcgaat attacatttc caacacctac accttgcaaa gagtttctaa tccatccggt     420
   aacttcgatt ctccaaatca tgatggtttg ggtgaaccta agttcaacgt tgatgatact     480
   gcttatacag cttcttgggg tagaccacaa aatgatggtc cagctttgag agcttacgct     540
   atttctagat acttgaacgc tgttgctaag cacaacaacg gtaaattatt attggccggt     600
   caaaacggta ttccttattc ttctgcttcc gatatctact ggaagattat taagccagac     660
   ttgcaacatg tttctactca ttggtctacc tctggttttg atttgtggga agaaaatcaa     720
   ggtactcatt tcttcaccgc tttggttcaa ttgaaggctt tgtcttacgg tattccattg     780
   tctaagacct acaatgatcc aggtttcact tcttggttgg aaaaacaaaa ggatgccttg     840
   aactcctaca ttaactcttc cggtttcgtt aactctggta aaaagcacat cgttgaatct     900
   ccacaattgt catctagagg tggtttggat tctgctactt atattgctgc cttgatcacc     960
   catgatatcg gtgatgatga tacttacacc ccattcaatg ttgataactc ctacgttttg    1020
   aactccttgt attacctatt ggtcgacaac aagaacagat acaagatcaa cggtaactac    1080
   aaagctggtg ctgctgttgg tagatatcct gaagatgttt acaacggtgt tggtacttct    1140
   gaaggtaatc catggcaatt ggctactgct tatgctggtc aaacttttta caccttggcc    1200
   tacaattcct tgaagaacaa gaagaacttg gtcatcgaaa agttgaacta cgacttgtac    1260
   aactccttca ttgctgattt gtccaagatt gattcttcct acgcttctaa ggattctttg    1320
   actttgacct acggttccga taactacaag aacgttatca agtccttgtt gcaattcggt 1380
   gactcattct tgaaggtttt gttggatcac atcgatgaca acggtcaatt gactgaagaa 1440
   atcaacagat acaccggttt tcaagctggt gcagtttctt tgacttggtc atctggttct 1500
   ttgttgtctg ctaatagagc cagaaacaag ttgatcgaat tattg 1545
<210> 18
   <211> 515
   <212> PRT
   <213> Saccharomycopsis fibuligera
<400> 18
   Met Ile Arg Leu Thr Val Phe Leu Thr Ala Val Phe Ala Ala Val Ala
   1 5 10 15
   Ser Cys Val Pro Val Glu Leu Asp Lys Arg Asn Thr Gly His Phe Gln
   20 25 30
   Ala Tyr Ser Gly Tyr Thr Val Ala Arg Ser Asn Phe Thr Gln Trp Ile
   35 40 45
   His Glu Gln Pro Ala Val Ser Trp Tyr Tyr Leu Leu Gln Asn Ile Asp
   50 55 60
   Tyr Pro Glu Gly Gln Phe Lys Ser Ala Lys Pro Gly Val Val Val Ala
   65 70 75 80
   Ser Pro Ser Thr Ser Glu Pro Asp Tyr Phe Tyr Gln Trp Thr Arg Asp
   85 90 95
   Thr Ala Ile Thr Phe Leu Ser Leu Ile Ala Glu Val Glu Asp His Ser
   100 105 110
   Phe Ser Asn Thr Thr Leu Ala Lys Val Val Glu Tyr Tyr Ile Ser Asn
   115 120 125
   Thr Tyr Thr Leu Gln Arg Val Ser Asn Pro Ser Gly Asn Phe Asp Ser
   130 135 140
   Pro Asn His Asp Gly Leu Gly Glu Pro Lys Phe Asn Val Asp Asp Thr
   145 150 155 160
   Ala Tyr Thr Ala Ser Trp Gly Arg Pro Gln Asn Asp Gly Pro Ala Leu
   165 170 175
   Arg Ala Tyr Ala Ile Ser Arg Tyr Leu Asn Ala Val Ala Lys His Asn
   180 185 190
   Asn Gly Lys Leu Leu Leu Ala Gly Gln Asn Gly Ile Pro Tyr Ser Ser
           195                 200                 205
   Ala Ser Asp Ile Tyr Trp Lys Ile Ile Lys Pro Asp Leu Gln His Val
       210                 215                 220
   Ser Thr His Trp Ser Thr Ser Gly Phe Asp Leu Trp Glu Glu Asn Gln
   225                 230                 235                 240
   Gly Thr His Phe Phe Thr Ala Leu Val Gln Leu Lys Ala Leu Ser Tyr
                   245                 250                 255
   Gly Ile Pro Leu Ser Lys Thr Tyr Asn Asp Pro Gly Phe Thr Ser Trp
               260                 265                 270
   Leu Glu Lys Gln Lys Asp Ala Leu Asn Ser Tyr Ile Asn Ser Ser Gly
           275                 280                 285
   Phe Val Asn Ser Gly Lys Lys His Ile Val Glu Ser Pro Gln Leu Ser
       290                 295                 300
   Ser Arg Gly Gly Leu Asp Ser Ala Thr Tyr Ile Ala Ala Leu Ile Thr
   305                 310                 315                 320
   His Asp Ile Gly Asp Asp Asp Thr Tyr Thr Pro Phe Asn Val Asp Asn
                   325                 330                 335
   Ser Tyr Val Leu Asn Ser Leu Tyr Tyr Leu Leu Val Asp Asn Lys Asn
               340                 345                 350
   Arg Tyr Lys Ile Asn Gly Asn Tyr Lys Ala Gly Ala Ala Val Gly Arg
           355                 360                 365
   Tyr Pro Glu Asp Val Tyr Asn Gly Val Gly Thr Ser Glu Gly Asn Pro
       370                 375                 380
   Trp Gln Leu Ala Thr Ala Tyr Ala Gly Gln Thr Phe Tyr Thr Leu Ala
   385                 390                 395                 400
   Tyr Asn Ser Leu Lys Asn Lys Lys Asn Leu Val Ile Glu Lys Leu Asn
                   405                 410                 415
   Tyr Asp Leu Tyr Asn Ser Phe Ile Ala Asp Leu Ser Lys Ile Asp Ser
               420                 425                 430
   Ser Tyr Ala Ser Lys Asp Ser Leu Thr Leu Thr Tyr Gly Ser Asp Asn
           435                 440                 445
   Tyr Lys Asn Val Ile Lys Ser Leu Leu Gln Phe Gly Asp Ser Phe Leu
   450 455 460
   Lys Val Leu Leu Asp His Ile Asp Asp Asn Gly Gln Leu Thr Glu Glu
   465 470 475 480
   Ile Asn Arg Tyr Thr Gly Phe Gln Ala Gly Ala Val Ser Leu Thr Trp
   485 490 495
   Ser Ser Gly Ser Leu Leu Ser Ala Asn Arg Ala Arg Asn Lys Leu Ile
   500 505 510
   Glu Leu Leu
   515
<210> 19
   <211> 70
   <212> DNA
   <213> Artificial Sequence
<220>
   <223> Amorce
<400> 19
   cgctccagaa ttagcggacc tcttgagcgg tgagcctctg gcaaagaaga gcataaccgc 60
   tagagtactt 70
<210> 20
   <211> 71
   <212> DNA
   <213> Artificial Sequence
<220>
   <223> Amorce
<400> 20
   cgctccagaa ttagcggacc tcttgagcgg tgagcctctg gcaaagaaga tgaagcttcg 60
   tacgctgcag g 71
<210> 21
   <211> 50
   <212> DNA
   <213> Artificial Sequence
<220>
   <223> Amorce
<400> 21
   tcactgtacg gtgagaacgt agatggtgtg cagcttgcaa attaaagcct 50
<210> 22
   <211> 70
   <212> DNA
   <213> Artificial Sequence
<220>
   <223> Amorce
<400> 22
   ctcaagaacg taggacgata actggttgga aagcgtaaac acggagtcaa cagcttgcaa    60
   attaaagcct                                                           70